(19) Europäisches Patentamt
European Patent Office
Office européen des brevets

(11) Veröffentlichungsnummer: **0 126 235**
**A1**

# (12) EUROPÄISCHE PATENTANMELDUNG

(21) Anmeldenummer: **84103069.5**

(22) Anmeldetag: **21.03.84**

(51) Int. Cl.³: **C 07 D 291/04**, C 07 F 7/10, C 07 F 9/65, A 01 N 43/00, A 01 N 55/00, A 01 N 57/36

(30) Priorität: **30.03.83 DE 3311636**

(43) Veröffentlichungstag der Anmeldung: **28.11.84**
**Patentblatt 84/48**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Jäger, Gerhard, Dr., Gellertstrasse 18, D-5090 Leverkusen (DE)**
Erfinder: **Fauss, Rudolf, Dr., Gerstenkamp 10, D-5000 Köln 80 (DE)**
Erfinder: **Findeisen, Kurt, Dr., In der Follmühle 10, D-5068 Odenthal 2 (DE)**
Erfinder: **Becker, Benedikt, Dr., Metzkausenerstrasse 14, D-4020 Mettmann (DE)**
Erfinder: **Homeyer, Bernhard, Dr., Obere Strasse 28, D-5090 Leverkusen 3 (DE)**

## (54) Neue fünfgliedrige stickstoffhaltige Heterocyclen, Verfahren zu ihrer Herstellung sowie ihre Verwendung als Schädlingsbekämpfungsmittel.

(57) Die vorliegende Erfindung betrifft neue fünfgliedrige stickstoffhaltige Heterocyclen der Formel (I)

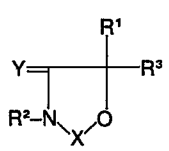

(I)

in welcher $R^1$, $R^2$, $R^3$, X, Y die in der Beschreibung angegebene Bedeutung besitzen.

Die Verbindungen der Formel I werden erhalten, indem man Verbindungen der Formel II

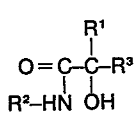

mit Verbindungen der Formel III

$$X^1Hal_2$$

umsetzt, oder indem man Verbindungen der Formel IV

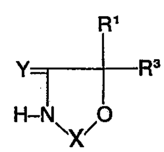

mit Verbindungen der Formel V

$$R^{14}Z$$

umsetzt, oder indem man Verbindungen der Formel VI

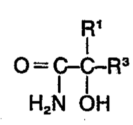

mit Verbindungen der Formel VII

$$R^{15}\text{–}NCO$$

(Fortsetzung nächste Seite)

EP 0 126 235 A1

umsetzt, und in Gegenwart einer Base erwärmt, oder indem man Verbindungen der Formel II mit Verbindungen der Formel IX

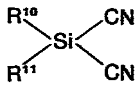

umsetzt, oder indem man Verbindungen der Formel X

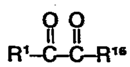

mit Verbindungen der Formel XI

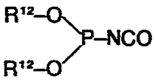

umsetzt, wobei in den Formeln die Reste die in der Beschreibung angegebene Bedeutung besitzen. Die Verbindungen der Formel I eignen sich als Schädlingsbekämpfungsmittel, insbesondere als Insektizide und Akarizide.

0126235



BAYER AKTIENGESELLSCHAFT 5090 Leverkusen, Bayerwerk
Konzernverwaltung RP
Patentabteilung

Rt/Kü-c
I b

Neue fünfgliedrige stickstoffhaltige Heterocyclen Verfahren zu ihrer Herstellung sowie ihre Verwendung als Schädlingsbekämpfungsmittel

Die vorliegende Erfindung betrifft neue fünfgliedrige stickstoffhaltige Heterocyclen, Verfahren zu ihrer Herstellung sowie ihre Verwendung als Schädlingsbekämpfungsmittel.

1,2,3-Oxathiazolidin-4-one sind bekannt aus FR-PS 2 315 422, Chemical Abstracts CA 86 : 106492c. Es ist daraus jedoch nichts über die Eingenschaften dieser Verbindungen als Schädlingsbekämpfungsmittel bekannt geworden. 1,2,3-Oxazaphospholidin-2-oxide sind bekannt aus J. Gen. Chem. USSR Band 43 I (1973) S. 256 - 259. Fs ist jedoch nichts über die Eigenschaften dieser Verbindungen als Schädlingsbekämpfungsmittel bekannt geworden.

Es ist bereits bekannt geworden, daß Carbamate wie 5,6-Dimethyl-2-dimethylamino-4-pyrimidinyl-dimethylcarbamat oder 1-Naphthyl-N-methylcarbamat insektizide Wirksamkeit besitzen (US-PS 3 493 574, 2 903 478).

Ihre Wirkung ist jedoch vor allem bei niedrigen Aufwandkonzentrationen nicht immer voll befriedigend.

1. Es wurden die neuen fünfgliedrigen sickstoffhaltigen Heterocyclen der Formel (I) gefunden

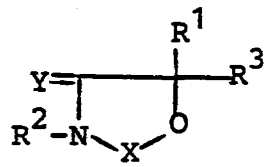

(I)

in welcher

$R^1$ für Alkyl, Alkenyl, Alkinyl, Cycloalkyl, Cycloalkenyl, Aryl, Heteroaryl, die gegebenenfalls substituiert sein können, steht

$R^2$ für Wasserstoff, ein Äquivalent eines anorganischen oder organischen Kations, Trialkylsilyl sowie für Alkyl, Cycloalkyl, Alkenyl, Alkinyl, Aryl, Aralkyl, Alkylcarbonyl, Arylcarbonyl, Alkoxycarbonyl, Aryloxycarbonyl, Alkylsulfenyl, Arylsulfenyl, Alkylsulfonyl, Arylsulfonyl, Alkylaminosulfonyl, Dialkylaminosulfonyl, Arylaminosulfonyl, Arylalkylaminosulfonyl, die gegebenenfalls substituiert sein können, sowie für Reste der Formel

$$- CO - NR^5R^6$$

steht, wobei

$R^5$ und $R^6$ unabhängig voneinander für Wasserstoff, Alkyl, Cycloalkyl, Aryl, Alkylaminocarbonyl, Arylaminocarbonyl, Alkylcarbonyl, Arylcarbonyl, Alkoxycarbonyl, Aryloxycarbonyl, Alkylsulfonyl, Arylsulfonyl, wobei diese Reste gegebenenfalls substituiert sein können stehen,

$R^3$ steht für Alkyl, Alkenyl, Alkinyl, Cycloalkyl, Cycloalkenyl, Aryl, Heteroaryl, die gegebenenfalls substituiert sind, sowie für -CN sowie

für den Rest $-\overset{\overset{O(S)}{\|}}{C}-R^4$

$R^4$ steht für Amino, -O-W wobei W für Wasserstoff, gegebenenfalls substituiertes Alkyl- oder Cycloalkyl, Aralkyl sowie für ein Äquivalent Alkali oder Erdalkali steht,

ferner steht $R^4$ für Alkylamino, Arylamino, Aralkylamino, Dialkylamino, Cycloalkylamino, Alkenylamino, Trialkylsilylamino, Trialkylsilylalkylamino, stickstoffhaltige gesättigte heterocyclische Reste die gegebenenfalls weitere Heteroatome enthalten, wobei die Reste gegebenenfalls substituiert sein können, ferner steht $R^4$ für Reste der Formel

$$-NHR^7$$

wobei

$R^7$ für Hydroxyl, Formyl, Alkylcarbonyl, Alkenylcarbonyl, Cycloalkenylcarbonyl, Arylcarbonyl, Amino, Alkylamino, Arylamino, Alkylarylamino, Dialkylamino, Alkylaminocarbonylamino (-NH-$\overset{O}{\overset{\|}{C}}$-NH-Alkyl), Alkylaminothiocarbonylamino (-NH-$\overset{S}{\overset{\|}{C}}$-NH-Alkyl), Arylaminocarbonylamino, Arylaminothiocarbonylamino, Alkylcarbonylamino (-NH-$\overset{O}{\overset{\|}{C}}$-Alkyl), Arylcarbonylamino, Alkylsulfonylaminocarbonylamino (-NH-$\overset{O}{\overset{\|}{C}}$-NH-$SO_2$-Alkyl), Arylsulfonylaminocarbonylamino, Alkylcarbonylaminocarbonylamino (-NH-$\overset{O}{\overset{\|}{C}}$-NH-$\overset{O}{\overset{\|}{C}}$-Alkyl), Arylcarbonylaminocarbonylamino wobei die Alkyl- oder Arylreste gegebenenfalls substituiert sein können, steht,

$R^4$ steht ferner für Reste der Formel

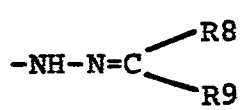

wobei

R8 für Alkyl oder Aryl die gegebenenfalls substituiert sein können, steht und

R9 für Wasserstoff oder Alkyl steht,

X für S, SO, $SO_2$, $>SiR^{10}R^{11}$, $>\overset{O}{\overset{\|}{P}}-O-R^{12}$ steht,

wobei

$R^{10}$ für Alkyl steht,

$R^{11}$ für Alkyl steht,

$R^{12}$ für Alkyl oder Aryl steht,

Y steht für O, S, $=N-R^{13}$

wobei

$R^{13}$ für Alkyl oder Aryl steht die gegebenenfalls substituiert sein können;

steht $R^1$ für Methyl oder Trifluormethyl darf $R^3$ nicht gleichzeitig für Methyl oder Trifluormethyl stehen.

Die fünfgliedrigen stickstoffhaltigen Heterocyclen der Formel I, liegen in Form der Gemische ihrer sterischen und optischen Isomere sowie in Form ihrer reinen Isomeren vor.

2. Es wurde ferner gefunden, daß man die fünfgliedrigen stickstoffhaltigen Heterocyclen der Formel I

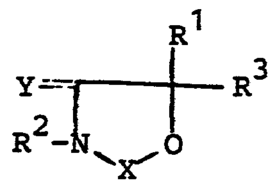

I

wobei

$R^1$ für Alkyl, Alkenyl, Alkinyl, Cycloalkyl, Cycloalkenyl, Aryl, Heteroalkyl, die gegebenenfalls substituiert sein können, steht

$R^2$ für Wasserstoff, ein Äquivalent eines anorganischen oder organischen Kations, Trialkylsilyl sowie für Alkyl, Cycloalkyl, Alkenyl, Alkinyl, Aryl, Aralkyl, Alkylcarbonyl, Arylcarbonyl, Alkoxycarbonyl, Aryloxycarbonyl, Alkylsulfenyl, Arylsulfenyl, Alkylsulfonyl, Arylsulfonyl, Alkylaminosulfonyl, Dialkylaminosulfonyl, Arylaminosulfonyl, Arylalkylaminosulfonyl, die gegebenenfalls substituiert sein können, sowie für Reste der Formel

$$-CO-NR^5R^6$$

steht, wobei

$R^5$ und $R^6$ unabhängig voneinander für Wasserstoff, Alkyl, Cycloalkyl, Aryl, Alkylaminocarbonyl,

Arylaminocarbonyl, Alkylcarbonyl, Arylcarbonyl, Alkoxycarbonyl, Aryloxycarbonyl, Alkylsulfonyl, Arylsulfonyl, wobei diese Reste gegebenenfalls substituiert sein können stehen,

$R^3$ steht für Alkyl, Alkenyl, Alkinyl, Cycloalkyl, Cycloalkenyl, Aryl, Heteroaryl, die gegebenenfalls substituiert sind, sowie für -CN sowie

für den Rest $-\overset{\overset{O(S)}{\|}}{C}-R_4$

$R^4$ steht für Amino, -OW wobei W für Wasserstoff, gegebenenfalls substituiertes Alkyl oder Cycloalkyl, Aralkyl sowie für ein Äquivalent Alkali oder Erdalkali steht,

ferner steht $R^4$ für Alkylamino, Arylamino, Aralkylamino, Dialkylamino, Cycloalkylamino, Alkenylamino, Trialkylsilylamino, Trialkylsilylalkylamino, stickstoffhaltige gesättigte heterocyclische Reste die gegebenenfalls weitere Heteroatome enthalten, wobei die Reste gegebenenfalls substituiert sein können, ferner steht $R^4$ für Reste der Formel

$$-NHR^7$$

wobei

$R^7$ für Hydroxyl, Formyl, Alkylcarbonyl, Alkenylcarbonyl, Cycloalkenylcarbonyl, Arylcarbonyl, Amino, Alkylamino, Arylamino, Alkylarylamino, Dialkylamino, Alkylaminocarbonylamino $(\text{-NH-}\overset{\overset{O}{\|}}{C}\text{-NH-Alkyl})$, Alkylaminothiocarbonylamino $(\text{-NH-}\overset{\overset{S}{\|}}{C}\text{-NH-Alkyl})$, Arylaminocarbonylamino, Arylaminothiocarbonylamino, Alkylcarbonylamino $(\text{-NH-}\overset{\overset{O}{\|}}{C}\text{-Alkyl})$, Arylcarbonylamino, Alkylsulfonylaminocarbonylamino $(\text{-NH-}\overset{\overset{O}{\|}}{C}\text{-NH-}SO_2\text{-Alkyl})$, Arylsulfonylaminocarbonylamino, Alkylcarbonylaminocarbonylamino $(\text{-NH-}\overset{\overset{O}{\|}}{C}\text{-NH-}\overset{\overset{O}{\|}}{C}\text{-Alkyl})$, Arylcarbonylaminocarbonylamino, wobei die Alkyl- oder Arylreste gegebenenfalls substituiert sein können, steht,

$R^4$ steht ferner für Reste der Formel

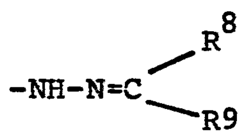

wobei

R8 für Alkyl oder Aryl die gegebenenfalls substituiert sein können steht und

R9 für Wasserstoff oder Alkyl steht,

X für S, SO, $SO_2$, CO, $>SiR^{10}R^{11}$, $>\overset{\overset{O}{\|}}{P}-R^{12}$ steht,

wobei

$R^{10}$ für Alkyl steht,

$R^{11}$ für Alkyl steht,

$R^{12}$ für Alkyl oder Aryl steht;

Y steht für O, S, $=N-R^{13}$

wobei

$R^{13}$ für Alkyl oder Aryl steht die gegebenenfalls substituiert sein können;

steht $R^1$ für Methyl oder Trifluormethyl darf $R^3$ nicht gleichzeitig für Methyl oder Trifluormethyl stehen;

erhält, indem man

a) für den Fall, daß Verbindungen der Formel I erhalten werden sollen, in welcher X für SO, S, $SiR^{10}R^{11}$ steht, Verbindungen der Formel II

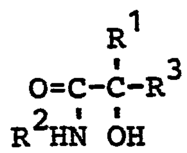

II

in welcher

$R^1$, $R^2$, $R^3$, die oben angegebene Bedeutung haben

mit Verbindungen der Formel III

$$X^1Hal_2 \qquad III$$

in welcher

Hal für Halogen steht,

$X^1$ für SO, $\rangle SiR^{10}R^{11}$, S steht,

umsetzt, und die entstehenden Verbindungen gegebenenfalls zur Salzbildung mit einer Base behandelt, oder

b) Verbindungen der Formel IV

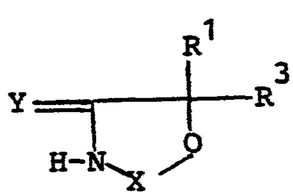

IV

in welcher

$R^1$, $R^3$, X und Y die oben angegebene Bedeutung haben

mit Verbindungen der Formel V

$$R^{14}-Z \qquad V$$

in welcher

$R^{14}$ für Trialkylsilyl, Alkyl, Cycloalkyl, Alkenyl, Alkinyl, Aralkyl, Aryl, Alkylcarbonyl, Arylcarbonyl, Alkoxycarbonyl, Aryloxycarbonyl, Alkylsulfenyl, Arylsulfenyl, Alkylsulfonyl, Arylsulfonyl, Alkylaminosulfonyl, Dialkylaminosulfonyl, Arylaminosulfonyl, Arylalkylaminosulfonyl, die gegebenenfalls substituiert sein können, sowie für Reste der Formel

$$-CO-NR^5R^6$$

steht, wobei

$R^5$ und $R^6$ unabhängig voneinander für Wasserstoff, Alkyl, Cycloalkyl, Aryl, Alkylaminocarbonyl, Arylaminocarbonyl, Alkylcarbonyl, Arylcarbonyl, Alkoxycarbonyl, Aryloxycarbonyl, Alkylsulfonyl,

Arylsulfonyl, wobei diese Reste gegebenenfalls substituiert sein können stehen,

Z für eine gegenüber einem aciden Wasserstoffatom reaktive Gruppe steht,

umsetzt, oder

c) für den Fall, daß Verbindungen der Formel I erhalten werden sollen, in denen X für $>SiR^{10}R^{11}$ steht, man Verbindungen der Formel II

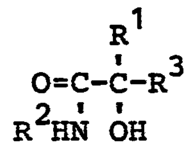

II

in welcher

$R^1$, $R^2$, $R^3$ die oben angegebene Bedeutung haben ($R^3$ jedoch nicht für -COOH stehen darf),

mit Verbindungen der Formel IX

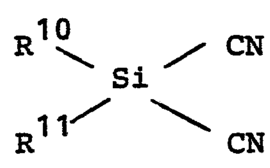

IX

in welcher

$R^{10}$ und $R^{11}$ die oben angegebene Bedeutung haben

umsetzt und die entstehenden Verbindungen gegebenenfalls zur Salzbildung mit einer Base behandelt, oder

d) für den Fall, daß Verbindungen der Formel I erhalten werden sollen in denen X für $>\overset{O}{\overset{\|}{P}}-OR^{12}$ und $R^2$ für den Rest $R^{12}$ steht man Verbindungen der Formel X

$$R^1-\overset{O}{\overset{\|}{C}}-\overset{O}{\overset{\|}{C}}-R^{16} \qquad X$$

in welcher

$R^1$ die oben angegebene Bedeutung besitzt und

$R^{16}$ für Amino, Alkylamino, Arylamino, Aralkylamino, Dialkylamino, Cycloalkylamino, Alkenylamino, Trialkylsilylamino, Trialkylsilylalkylamino,stickstoffhaltige gesättigte heterocyclische Reste die gegebenenfalls weitere Heteroatome enthalten, wobei die Reste gegebenenfalls substituiert sein können, ferner steht $R^4$ für Reste der Formel

$-NHR^7$

wobei

$R^7$ für Hydroxyl, Formyl, Alkylcarbonyl, Alkenylcarbonyl, Cycloalkenylcarbonyl, Arylcarbonyl, Amino, Alkylamino, Arylamino, Alkylarylamino, Dialkylamino, Alkylaminocarbonylamino ($-NH-\overset{O}{\overset{\parallel}{C}}-NH-Alkyl$), Alkylaminothiocarbonylamino ($-NH-\overset{S}{\overset{\parallel}{C}}-NH-Alkyl$), Arylaminocarbonylamino, Arylaminothiocarbonylamino, Alkylcarbonylamino ($-NH-\overset{O}{\overset{\parallel}{C}}-Alkyl$), Arylcarbonylamino, Alkylsulfonylaminocarbonylamino ($-NH-\overset{O}{\overset{\parallel}{C}}-NH-SO_2-Alkyl$), Arylsulfonylaminocarbonylamino, Alkylcarbonylaminocarbonylamino, ($-NH-\overset{O}{\overset{\parallel}{C}}-NH-\overset{O}{\overset{\parallel}{C}}-Alkyl$),

Arylcarbonylaminocarbonylamino steht, wobei die Alkyl- oder Arylreste gegebenenfalls substituiert sein können,

$R^{16}$ steht ferner für Reste der Formel

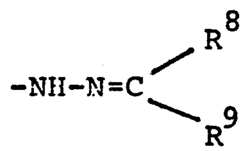

wobei

$R^8$ für Alkyl oder Aryl die gegebenenfalls substituierte sein können, steht und

$R^9$ für Wasserstoff oder Alkyl steht,

mit Verbindungen der Formel XI

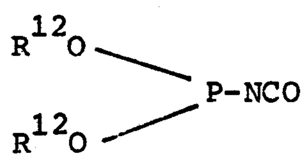

XI

in welcher

$R^{12}$ die oben angegebene Bedeutung hat

umsetzt, oder

e) für den Fall, daß Verbindungen der Formel I erhalten werden sollen, in welcher X für $>SO_2$ steht, man Verbindungen der Formel I in welcher X für SO steht mit Oxidationsmitteln wie Peroxiden und Persäuren umsetzt.

Überraschenderweise zeigen die erfindungsgemäßen fünfgliedrigen stickstoffhaltigen Heterocyclen eine erheblich höhere insektizide Wirkung als die aus dem Stand der Technik bekannten Carbamate der gleichen Wirkungsrichtung wie 5,6-Dimethyl-2-dimethylamino-4-pyrimidinyl-dimethylcarbamat oder 1-Naphthyl-N-methylcarbamat (US-PS 3 493 574 und US-PS 2 903 478).

Außerdem weisen die neuen fünfgliedrigen stickstoffhaltigen Heterocyclen eine günstige Toxizität und eine wurzelsystemische Wirkung auf.

Bevorzugt sind Verbindungen der Formel I in welcher

$R^1$ für $C_{1-7}$-Alkyl, $C_{2-7}$-Alkenyl, $C_{3-7}$-Cycloalkyl, $C_{5-8}$-Cycloalkenyl steht, die gegebenenfalls gleich oder verschieden durch einen oder mehrere der folgenden Reste substituiert sein können:

Halogen, insbesondere Fluor, Chlor, Brom, $C_{1-4}$-Alkoxy insbesondere Methoxy oder Ethoxy, Carboxyl, Carbalkoxy insbesondere Methoxycarbonyl oder Ethoxycarbonyl, Phenyl, Phenoxy, Thiophenyl wobei die Phenylringe durch Halogen oder Alkyl substituiert sein können;

$R^1$ steht ferner für Phenyl das gegebenenfalls gleich oder verschieden durch einen oder mehrere der folgenden Reste substituiert sein kann: Halogen, insbesondere Chlor, Brom, Fluor, Nitro, Amino, OH, CN, $C_{1-4}$-Alkyl insbesondere Methyl, $C_{1-4}$-Halogenalkyl insbesondere Trifluormethyl, Trichlormethyl, Pentafluorethyl, $C_{1-4}$-Alkoxy, Methylendioxy, Ethylendioxy, $C_{1-4}$-Halogenalkoxy, insbesondere Trifluormethoxy, Pentafluorethoxy, Difluormethylendioxy, halogensubstituiertes Ethylendioxy, $C_{1-4}$-Alkylthio, $C_{1-4}$-Halogenalkylthio insbesondere Trifluormethylthio, $C_{2-8}$-Alkoxyalkyl, $C_{2-8}$-Halogenalkoxyalkyl, $C_{1-4}$-Alkylsulfonyl insbesondere Methylsulfonyl, $C_{1-4}$-Halogenalkylsulfonyl, Carboxyl, Carbalkoxy insbesondere Methoxycarbonyl, sowie für den Rest $C_{1-4}$-Alkoxy-N=CH-, insbesondere $CH_3$-O-N=CH-, ferner für Phenyl, Phenyloxy, Thiophenyl die gegebenenfalls durch Halogen oder $C_{1-4}$-Alkyl substituiert sein können, sowie für Carboxylalkoxy mit 2 - 4 C-Atomen wie Carboxymethoxy,

$R^1$ steht ferner für Heteroaryl wie Pyridinyl, Pyrimidinyl, Triazinyl, Isoxazolyl, Thiazolyl, Oxadiazolyl, Imidazolyl, Triazolyl, Furanyl, Thiophenyl, die gegebenenfalls ein oder mehrfach gleich oder verschieden durch Halogen insbesondere Chlor, $C_{1-4}$-Alkyl, insbesondere Methyl, Ethyl, $C_{1-4}$-Alkoxy, insbesondere Methoxy, Ethoxy substituiert sein können.

$R^1$ steht außerdem bevorzugt für

o-, m-, p-Chlorphenyl, 2,3-Dichlorphenyl, 3,4-Dichlorophenyl, 3,5-Dichlorphenyl, 2,4-Dichlorphenyl, 2,5-Dichlorphenyl, 2,6-Dichlorphenyl, o-, m-, p-Nitrophenyl, o-Chloro-methylphenyl, 2,6-Dimethoxyphenyl, o-, m-, p-Trifluormethylphenyl, o-, m-, p-Methoxyphenyl, o-, m-, p-Tolyl, o-, m-, p-Trifluormethoxyphenyl, o-, m-, p-Fluorphenyl, Cyclohexyl, Benzyl, Butyl, tert.-Butyl, Fluor-tert.-butyl, Chlor-tert.-butyl, Difluor-tert.-butyl, Trichlormethyl, 3-Jodphenyl, Biphenyl, 4-Trimethylsilyloxyphenyl, 4-Chlor-3-nitrophenyl, 3-Chlor-4-nitrophenyl, 3,4,5-Trichlorphenyl, 3,5-Dichlor-4-fluorphenyl, 4-Difluormethylphenyl, 3-Nitro-4-fluorphenyl, 3-Fluor-4-nitrophenyl, 3-Trifluormethyl-4-chlorphenyl, 4-Trifluormethyl-3-chlorphenyl, 3-Chlor-5-trifluormethylphenyl, 3,4-(Di(trifluormethyl), 3,5-Di(trifluormethyl)-phenyl, 3-Trifluormethyl-4,5-dichlorphenyl, 4-Trifluormethyl-3,5-dichlorphenyl, 4-Trifluormethoxy-3-nitrophenyl, 4-Trifluormethoxy-3-bromphenyl, 4-Nitro-3-trifluormethoxyphenyl, 4-Brom-3-trifluormethoxyphenyl, 3-Nitro-4-trifluormethoxy-5-chlorphenyl, 4-Methoxy-3,5-dichlorphenyl, 4-Methyl-3,5-dichlorphenyl, 4-Fluor-3-bromphenyl, 4-Brom-3-fluorphenyl, 4-Chlor-3-methylphenyl, 4-Trifluormethylmercaptophenyl, 4-Trifluormethoxy-3-chlorphenyl, 3-Trifluormethyl-4-chlorphenyl, 4-Chlordifluormethoxy-3-chlorphenyl, 4-Fluor-3-chlorphenyl, Pentafluorphenyl, 4-Fluor-3,5-dibromphenyl, 4-Fluor-3-chlor-5-bromphenyl, 4-Chlor-3,5-dibromphenyl, 4-Brom-3,5-dichlorphenyl, 3-Brom-4,5-dichlorphenyl, 3,4,5-Trifluorphenyl, 3,4,5-Tribromphenyl.

Bevorzugt steht in der Formel I

$R^2$ für Wasserstoff, ein Äquivalent eines Alkali, Erdalkali oder gegebenenfalls substituierten Ammonium-Kations, Trialkylsilyl mit 1 - 4 C-Atomen im Alkylteil, $C_{1-4}$-Alkyl, $C_{1-4}$-Alkylcarbonyl, Phenyl, Benzoyl, die gegebenenfalls gleich oder verschieden durch einen oder mehrere der folgenden Reste (A) substituiert sein können: (A) steht für Halogen, insbesondere Chlor, Brom, Fluor, Nitro, Amino, CN, $C_{1-4}$-Alkyl insbesondere Methyl, $C_{1-4}$-Halogenalkyl insbesondere Trifluormethyl, Pentafluorethyl, $C_{1-4}$-Alkoxy, $C_{1-4}$-Halogenalkoxy, insbesondere Trifluormethoxy, Pentafluorethoxy, Methylendioxy, Ethylendioxy, Difluormethylendioxy, halogensubstituierte Ethylendioxy wie Trifluorethylendioxy, $C_{1-4}$-Alkylthio, $C_{1-4}$-Halogenalkylthio insbesondere Trifluormethylthio, $C_{2-8}$-Alkoxyalkyl, $C_{2-8}$-Halogenalkoxyalkyl, $C_{1-5}$-Alkylsulfenyl, Phenylsulfenyl die gegebenenfalls substituiert sein können, $C_{1-4}$-Alkylsulfonyl insbesondere Methylsulfonyl, $C_{1-4}$-Halogenalkylsulfonyl, Carbalkoxy insbesondere Methoxycarbonyl, sowie für den Rest $C_{1-4}$-Alkoxy-N=CH-, insbesondere $CH_3$-O-N=CH-, ferner für Phenyl, Phenyloxy, Thiophenyl die gegebenenfalls durch Halogen oder $C_{1-4}$-Alkyl substituiert sein können, sowie für Carboxyalkoxy mit 2 - 4 C-Atomen, wie Carboxymethoxy;

$R^2$ steht ferner für $C_{1-4}$-Alkoxycarbonyl, Phenoxycarbonyl, das gegebenenfalls durch einen oder mehrere

der Reste (A) substituiert sein kann, $C_{1-4}$-Alkylsulfonyl, Phenylsulfonyl das gegebenenfalls durch einen oder mehrere der Reste (A) substituiert sein kann, $C_{1-4}$-Alkylaminosulfonyl, $C_{1-5}$-Alkylsulfenyl, Phenylsulfenyl die gegebenenfalls substituiert sein können, Di-$C_{1-4}$-alkylaminosulfonyl, Phenylaminosulfonyl das gegebenenfalls durch einen oder mehrere der Reste (A) substituiert sein kann, Phenyl-$C_{1-4}$-alkyl-aminosulfonyl sowie für Reste der Formel

$$\overset{O}{\underset{}{\overset{\|}{-C}}}-NR^5R^6$$

wobei

$R^5$ und $R^6$ unabhängig voneinander für Wasserstoff, $C_{1-20}$-Alkyl, $C_{3-6}$-Cycloalkyl, Phenyl das gegebenenfalls durch einen oder mehrere der Reste (A) substituiert sein kann, Phenylcarbonyl, das gegebenenfalls durch einen oder mehrere der Reste (A) substituiert sein kann, $C_{1-10}$-Alkylcarbonyl, $C_{1-4}$-Alkylaminocarbonyl, $C_{1-10}$-Alkoxycarbonyl, Phenylaminocarbonyl, Phenylsulfonyl die gegebenenfalls durch einen oder mehrere der Reste (A) substituiert sein können, stehen.

Bevorzugt stehen in Formel I

$R^3$ für die bei $R^1$ angegebenen Reste sowie für CN oder für den Rest $-COR^4$

R4 steht vorzugsweise für Amino, C1-8-Amino, $C_{1-8}$-Alkylamino, Di-$C_{1-8}$-alkylamino, die gegebenenfalls durch Hydroxyl, $C_{1-4}$-Alkoxy substituiert sein können, Phenylamino das gegebenenfalls durch einen oder mehrere der oben angegebenen Reste (A) substituiert sein kann, $C_{5-6}$-Cycloalkylamino, einen gesättigten Heterocyclus mit 5 - 6 Ring C-Atomen wie Morpholin, Tri-$C_{1-4}$-alkylsilylamino, sowie für Reste der Formel $-NHR^7$

wobei

$R^7$ für $C_{1-4}$-Alkylcarbonyl, $C_{2-4}$-Alkenylcarbonyl, $C_{5-8}$-Cycloalkenylcarbonyl, wie Acetyl, $C_{1-4}$-Alkylamino wie Methylamino oder t-Butylamino, Phenylamino das gegebenenfalls durch einen oder mehrere der oben angegebenen Reste (A) subsituiert sein kann,

$C_1$-$C_4$-Alkylaminocarbonylamino ($-NH-\overset{O}{\overset{\|}{C}}-NH-Alkyl$), Phenylaminocarbonylamino ($-NH-\overset{O}{\overset{\|}{C}}-NH-Phenyl$) das gegebenenfalls durch einen oder mehrere der Reste (A) substituiert sein kann, $C_{1-4}$-Alkylcarbonylamino ($-NH-\overset{O}{\overset{\|}{C}}-Alkyl$), Benzoylamino das gegebenenfalls durch einen oder mehrere der Reste (A) substituiert sein kann, steht,

$R^4$ steht ferner für einen Rest der Formel

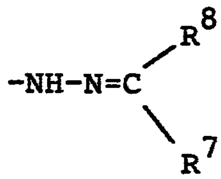

wobei

$R^8$ für $C_{1-4}$-Alkyl insbesondere Methyl, Phenyl das gegebenenfalls durch einen oder mehrere der Reste (A) substituiert sein kann, steht,

$R^9$ für Wasserstoff oder $C_{1-4}$-Alkyl steht.

Bevorzugt steht in Formel I

Y für O und

X für SO und $\rangle SiR^{10}R^{11}$

wobei

$R^{10}$ und $R^{11}$ für $C_{1-4}$-Alkyl insbesondere für Methyl stehen.

Besonders bevorzugt sind Verbindungen der Formel I

in welcher

$R^1$ für $C_{1-6}$-Alkyl das gegebenenfalls durch Halogen insbesondere Chlor oder $C_{1-4}$-Alkoxycarbonyl insbe-

sondere Ethoxycarbonyl, Phenoxy substituiert ist, wobei der Phenoxyrest gegebenenfalls durch Halogen wie Chlor oder Fluor oder durch Methyl substituiert ist, $C_{5-6}$-Cycloalkyl, für gegebenenfalls durch Carboxyl substituiertes $C_{2-4}$-Alkenyl, ferner für Phenyl, das gegebenenfalls durch Halogen, insbesondere Fluor oder Chlor, $C_{1-4}$-Alkyl, insbesondere Methyl, $C_{1-4}$-Alkoxy, $C_{1-4}$-Halogenalkoxy, $C_{1-4}$-Halogenalkyl, $NH_2$, $Ch_3O$-N=CH-, oder Nitro substituiert ist, steht und

$R^2$ für Wasserstoff, $C_{1-6}$-Alkyl, $C_{2-4}$-Alkenyl, $C_{2-4}$-Alkinyl für ein Äquivalent eines Kations aus der Reihe Natrium, Kalium, Calcium, Ammonium, Tri-($C_{1-4}$-alkyl)-ammonium, Morpholinyl, ferner für $C_{1-4}$-Alkylsulfenyl, $C_{1-4}$-Halogenalkylsulfenyl, Phenylsulfenyl das gegebenenfalls durch Halogen oder $C_{1-4}$-Alkyl substituiert ist, Phenyl, das gegebenenfalls durch Halogen oder $C_{1-4}$-Alkyl substituiert ist steht, und

$R^3$ für die bei $R^1$ angegebenen Reste sowie für den Rest $COR^4$ steht, wobei $R^4$ bevorzugt für Amino, $C_{1-4}$-Alkylamino, $C_{1-4}$-Alkoxy steht,

X für SO,$>Si(CH_3)_2$ steht und

Y für O steht.

Ganz besonders zu erwähnen sind Verbindungen der allgemeinen Formel I in welcher

$R^1$ für $C_{1-4}$-Alkyl insbesondere Methyl, t-Butyl, Methylcarbonyl-$C_{1-4}$-alkoxy wie Methylcarbonylethoxy, $C_{1-4}$-Halogenalkyl, insbesondere Chlormethyl, Phenoxymethyl das gegebenenfalls durch Chlor, Fluor, Methyl substituiert ist, Phenyl das gegebenenfalls ein bis dreifach gleich oder verschieden durch Halogen, insbesondere Fluor, Chlor, Brom, Jod, $C_{1-4}$-Alkyl insbesondere Methyl, Nitro, $C_{1-4}$-Alkoxy substituiert ist steht,

$R^2$ für Wasserstoff, Natrium, $C_{1-4}$-Alkyl insbesondere Methyl, Ethyl, $C_{1-4}$-Halogenalkyl, gegebenenfalls halogensubstituiertes $C_{1-4}$-Alkylsulfenyl, insbesondere Chlorfluormethylsulfenyl, $C_{1-4}$-Alkylcarbonyl, insbesondere Methylcarbonyl gegebenenfalls durch Halogen substituiertes Phenyl, Phenylcarbonyl oder Phenylsulfenyl, $C_{1-4}$-Alkenyl insbesondere Allyl, $C_{1-4}$-Alkinyl, insbesondere Propargyl, Trialkylsilyl insbesondere Trimethylsilyl

sowie für $-\overset{O}{\overset{\|}{C}}-NR^5R^6$ steht, wobei $R^5$ für Wasserstoff steht und $R^6$ für gegebenenfalls durch Halogen substituiertes Phenyl oder Phenylcarbonyl oder für $C_{1-4}$-Alkyl oder $C_{1-4}$-Alkylcarbonyl steht,

$R^3$ für die bei $R^1$ angegebenen Reste sowie für den Rest $COR^4$ steht wobei $R^4$ für Amino steht,

X für SO, und $Si(CH_3)_2$ steht und

Y für O steht.

Besonders seien genannt Verbindungen der Formel I in welcher

$R^1$ für t-Butyl, Chlormethyl, $C_{1-4}$-Alkoxycarbonylmethyl, gegebenenfalls durch Chlor oder Fluor substituiertes Phenoxymethyl sowie für Phenyl steht das gegebenenfalls ein oder mehrfach durch Chlor, Methyl, Nitro, Trifluormethyl substituert ist und

$R^2$ für Wasserstoff, Natrium, Methyl, Ethyl, Phenyl, 3,4-Dichlorphenyl, Propargyl, Trihalogenmethylsulfenyl, Tri($C_{1-4}$-alkyl)ammonium, Morpholinyl steht,

$R^3$ für die bei $R^1$ angegebenen Reste sowie für $COR^4$ steht wobei $R^4$ für Amino steht.

Im einzelnen seien die folgenden Verbindungen der Formel I genannt:

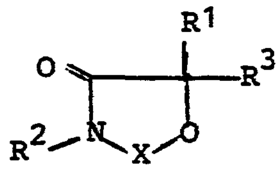

| $R^1$ | $R^2$ | $R^3$ | X |
|---|---|---|---|
| 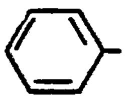 | H | $CONH_2$ | SO |
| 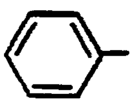 | $CH_3$ | $CONH_2$ | SO |
| 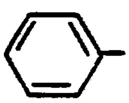 | $H_2C{=}CH{-}CH_2{-}$ | $CONH_2$ | SO |
| 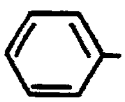 | $CH_3CO-$ | $CONH_2$ | SO |
| 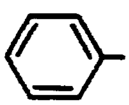 | $CH_3{-}NH{-}CO-$ | $CONH_2$ | SO |
| 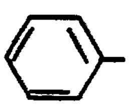 | H | $CCl_3$ | SO |
| 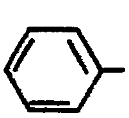 | H | $-CH_2COOC_2H_5$ | SO |
| 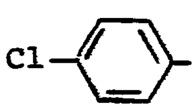 | H | $CONH_2$ | SO |

| $R^1$ | $R^2$ | $R^3$ | X |
|---|---|---|---|
| 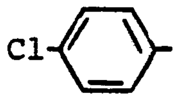 | $Na^+$ | $CONH_2$ | SO |
| 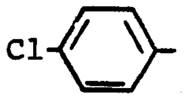 | $CH_3$ | $CONH_2$ | SO |
| 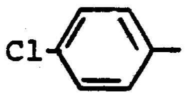 | $C_2H_5$ | $CONH_2$ | SO |
| 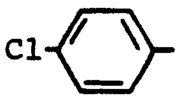 | 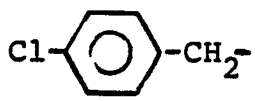 | $CONH_2$ | SO |
| 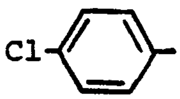 | $CH_3SO_2$- | $CONH_2$ | SO |
| 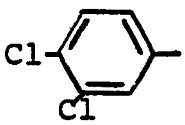 | H | $CONH_2$ | SO |
| 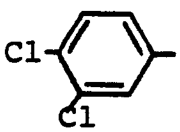 | $Na^+$ | $CONH_2$ | SO |
| 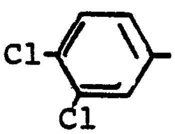 | $(C_2H_5)_3NH^+$ | $CONH_2$ | SO |
| 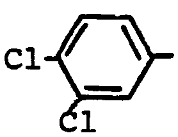 | $CH_3$ | $CONH_2$ | SO |
| 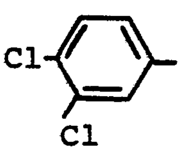 | $C_2H_5$ | $CONH_2$ | SO |

| $R^1$ | $R^2$ | $R^3$ | X |
|---|---|---|---|
| 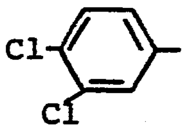 | $n-C_3H_7$ | $CONH_2$ | SO |
| 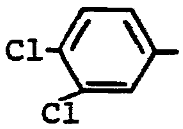 | $H_2C=CH-CH_2-$ | $CONH_2$ | SO |
| 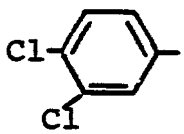 | $HC\equiv C-CH_2-$ | $CONH_2$ | SO |
| 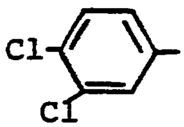 | $CH_3CO-$ | $CONH_2$ | SO |
| 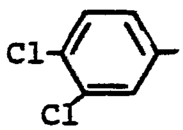 | $CH_3NH-CO-$ | $CONH_2$ | SO |
| 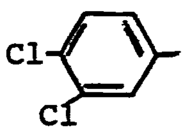 | $CCl_3S-$ | $CONH_2$ | SO |
| 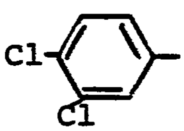 | $CFCl_2S-$ | $CONH_2$ | SO |
| 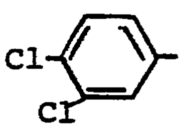 | 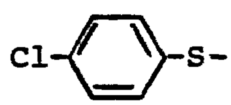 | $CONH_2$ | SO |
| 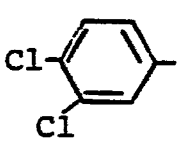 | 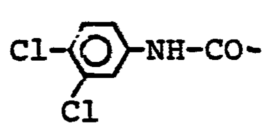 | $CONH_2$ | SO |
| 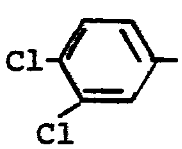 | 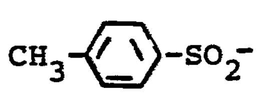 | $CONH_2$ | SO |

| $R^1$ | $R^2$ | $R^3$ | X |
|---|---|---|---|
| 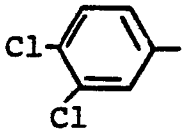 | 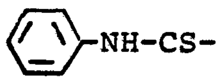 | $CONH_2$ | SO |
| 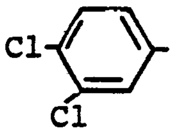 | 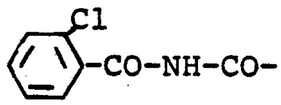 | $CONH_2$ | SO |
| 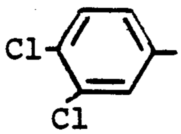 | 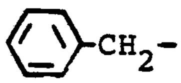 | $CONH_2$ | SO |
| 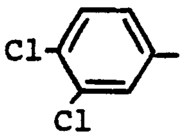 | 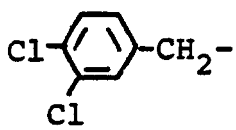 | $CONH_2$ | SO |
| 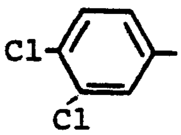 | $n-C_4H_9-NH-CO-$ | $CONH_2$ | SO |
| 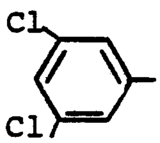 | H | $CONH_2$ | SO |
| 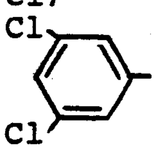 | $Na^+$ | $CONH_2$ | SO |
| 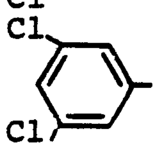 | $CH_3$ | $CONH_2$ | SO |
| 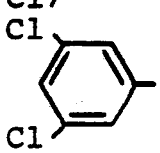 | $HC\equiv C-CH_2-$ | $CONH_2$ | SO |
| 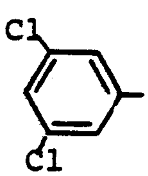 | $(CH_3)_2CH-$ | $CONH_2$ | SO |

| $R^1$ | $R^2$ | $R^3$ | X |
|---|---|---|---|
| 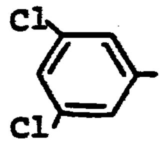 | $CCl_3S$- | $CONH_2$ | SO |
| 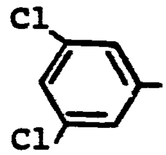 | 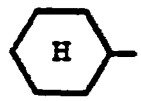 | $CONH_2$ | SO |
| 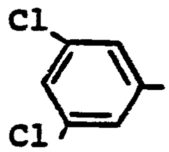 | n-$C_{12}H_{25}$ | $CONH_2$ | SO |
| 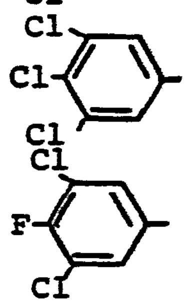 | H | $CONH_2$ | SO |
| | H | $CONH_2$ | SO |
| 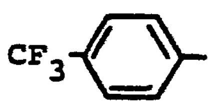 | H | $CONH_2$ | SO |
| 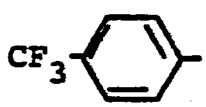 | $CH_3$ | $CONH_2$ | SO |
| 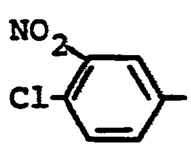 | H | $CONH_2$ | SO |
| 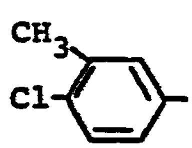 | H | $CONH_2$ | SO |
| 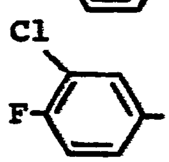 | H | $CONH_2$ | SO |
| 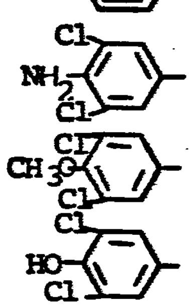 | H | $CONH_2$ | SO |
| | H | $CONH_2$ | SO |
| | H | $CONH_2$ | SO |

| $R^1$ | $R^2$ | $R^3$ | X |
|---|---|---|---|
| 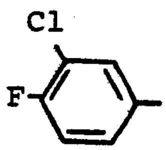 | $CH_3$ | $CONH_2$ | SO |
| 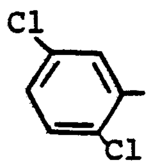 | H | $CONH_2$ | SO |
| 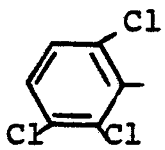 | H | $CONH_2$ | SO |
| 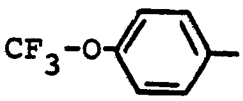 | H | $CONH_2$ | SO |
| 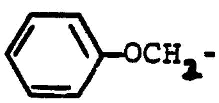 | H | $CONH_2$ | SO |
| 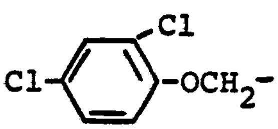 | H | $CONH_2$ | SO |
| $ClCH_2$ | H | $t-C_4H_9$ | SO |
| $BrCH_2$ | H | 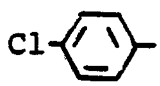 | SO |
| 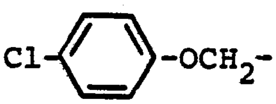 | H | $t-C_4H_9$ | SO |
| 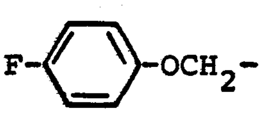 | H | $t-C_4H_9$ | SO |

| $R^1$ | $R^2$ | $R^3$ | X |
|---|---|---|---|
| 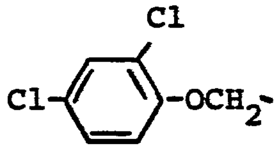 | H | t-$C_4H_9$ | SO |
| 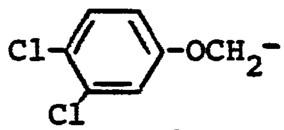 | H | t-$C_4H_9$ | SO |
| 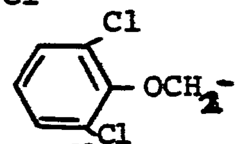 | H | t-$C_4H_9$ | SO |
| 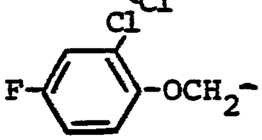 | H | t-$C_4H_9$ | SO |
| 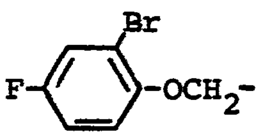 | H | t-$C_4H_9$ | SO |
| 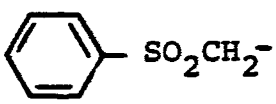 | H | t-$C_4H_9$ | SO |
| 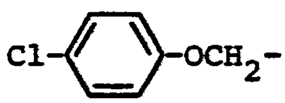 | $CH_3$ | t-$C_4H_9$ | SO |
| 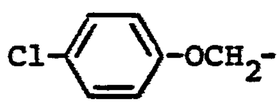 | $C_2H_5$ | t-$C_4H_9$ | SO |
| 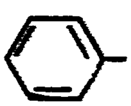 | H | 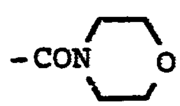 | SO |

| $R^1$ | $R^2$ | $R^3$ | X |
|---|---|---|---|
| 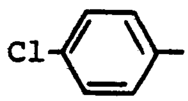 | H | $CONH_2$ | $>Si(CH_3)_2$ |
| 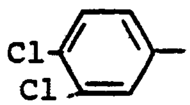 | H | $CONH_2$ | $>Si(CH_3)_2$ |
| 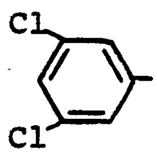 | H | $CONH_2$ | $>Si(CH_3)_2$ |
| 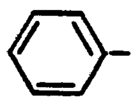 | H | $CONH_2$ | $>Si(CH_3)_2$ |
| 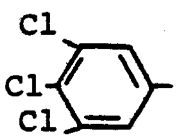 | H | $CONH_2$ | $>Si(CH_3)_2$ |
| 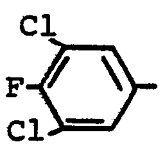 | H | $CONH_2$ | $>Si(CH_3)_2$ |
| 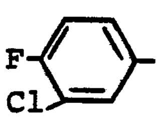 | H | $CONH_2$ | $>Si(CH_3)_2$ |
| 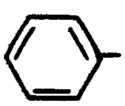 | $C_2H_5$ | $COOCH_3$ | $-\overset{O}{\overset{\|}{P}}-OC_2H_5$ |
| 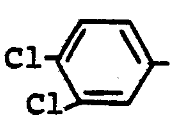 | $C_2H_5$ | $CONH_2$ | $-\overset{O}{\overset{\|}{P}}-OC_2H_5$ |
| 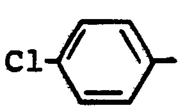 | $C_2H_5$ | $CONH_2$ | $-\overset{O}{\overset{\|}{P}}-OC_2H_5$ |

| $R^1$ | $R^2$ | $R^3$ | X |
|---|---|---|---|
| 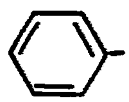 | $C_2H_5$ | $CONH_2$ | 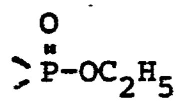 |
| 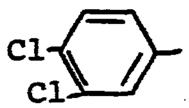 | H | $CONH_2$ | S |
| 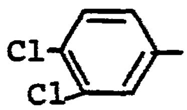 | H | $CONH_2$ | $SO_2$ |
| 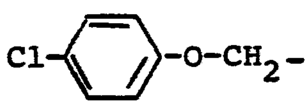 | H | $^tC_4H_9$ | $SO_2$ |

Weiter seien die folgenden Verbindungen genannt:

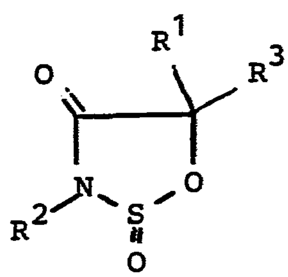

| $R^1$ | $R^2$ | $R^3$ |
|---|---|---|
| t-Butyl | H | $3,4-Cl_2-C_6H_3OCH_2-$ |
| t-Butyl | $CH_3$ | " |

| $R^1$ | $R^2$ | $R^3$ |
|---|---|---|
| t-Butyl | $C_2H_5$ | $3,4-Cl_2-C_6H_3OCH_2-$ |
| t-Butyl | $C_3H_7$ | " |
| t-Butyl | $n-C_4H_9$ | " |
| t-Butyl | $i-C_3H_7$ | " |
| t-Butyl | $HC{\equiv}C-CH_2-$ | " |
| t-Butyl | $H_2C{=}CH-CH_2-$ | " |
| t-Butyl | 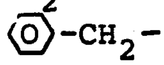 | " |
| t-Butyl | 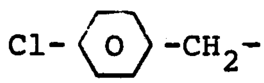 | " |
| t-Butyl | 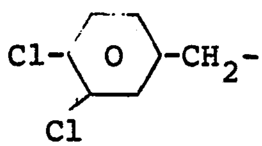 | " |
| t-Butyl | 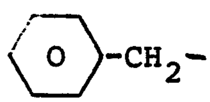 | $4-Cl-C_6H_4OCH_2-$ |
| t-Butyl | 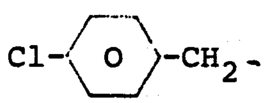 | " |
| t-Butyl | $i-C_3H_7$ | " |
| t-Butyl | $n-C_4H_9$ | " |
| t-Butyl | $n-C_8H_{17}$ | " |
| t-Butyl | 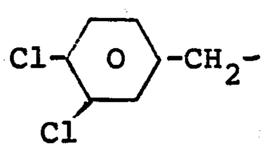 | " |

| $R^1$ | $R^2$ | $R^3$ |
|---|---|---|
| $4-Cl-C_6H_4-$ | H | $4-Cl-C_6H_4OCH_2-$ |
| " | $CH_3$ | " |
| " | $C_2H_5$ | " |
| " | $n-C_3H_7$ | " |
| " | $i-C_3H_7$ | " |
| " | $HC\equiv C-CH_2-$ | " |
| " | $H_2C=CH-CH_2-$ | " |
| " | $n-C_4H_9$ | " |
| " | 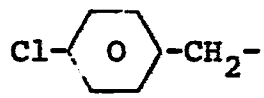 | " |
| " | 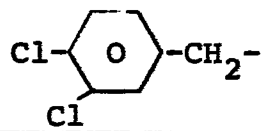 | " |
| " | H | $3,4-Cl_2-C_6H_3OCH_2-$ |
| " | $CH_3$ | " |
| " | $C_2H_5$ | " |
| " | $C_3H_7$ | " |
| " | $i-C_3H_7$ | " |
| " | $n-C_4H_9$ | " |
| " | $H_2C=CH-CH_2$ | " |
| " | $HC\equiv C-CH_2$ | " |
| " | 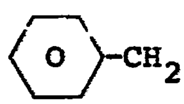 | " |
| $3,4-Cl_2-C_6H_3$ | H | $4-Cl-C_6H_4OCH_2-$ |
| " | $CH_3$ | " |
| " | $C_2H_5$ | " |

| $R^1$ | $R^2$ | $R^3$ |
|---|---|---|
| $3,4-Cl_2-C_6H_3-$ | $n-C_3H_7$ | $4-Cl-C_6H_4OCH_2-$ |
| " | $i-C_3H_7$ | " |
| " | $HC\equiv C-CH_2-$ | " |
| " | $H_2C=CH-CH_2-$ | " |
| " | 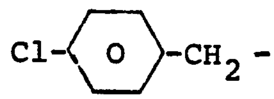 | " |
| " | 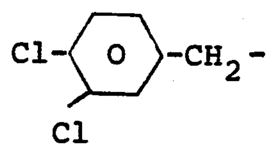 | " |
| " | H | $3,4-Cl_2-C_6H_3OCH_2-$ |
| " | $CH_3$ | " |
| " | $C_2H_5$ | " |
| " | $C_3H_7$ | " |
| " | $i-C_3H_7$ | " |
| " | $HC\equiv C-CH_2-$ | " |
| " | $H_2C=CH-CH_2-$ | " |
| " | $n-C_4H_9$ | " |
| " | 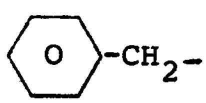 | " |
| " | $n-C_6H_{13}$ | " |

| $R^1$ | $R^2$ | $R^3$ |
|---|---|---|
| $4-Cl-C_6H_4-$ | $CH_3$ | $CONH_2$ |
| " | $C_2H_5$ | " |
| " | $HC\equiv C-CH_2-$ | " |
| " | $H_2C=CH-CH_2-$ | " |

Verfahren 2a:

Die Umsetzung der Verbindungen der Formeln II und III erfolgt gegebenenfalls in Gegenwart von Katalysatoren. Sie erfolgt gegebenenfalls in Gegenwart von Verdünnungsmitteln. Sie läßt sich z.B. durch folgende Formelschemata beschreiben:

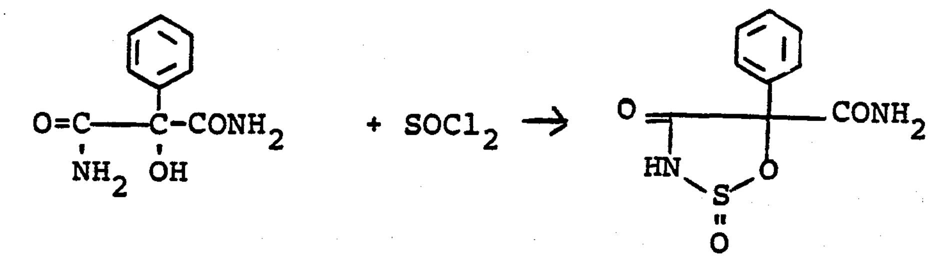


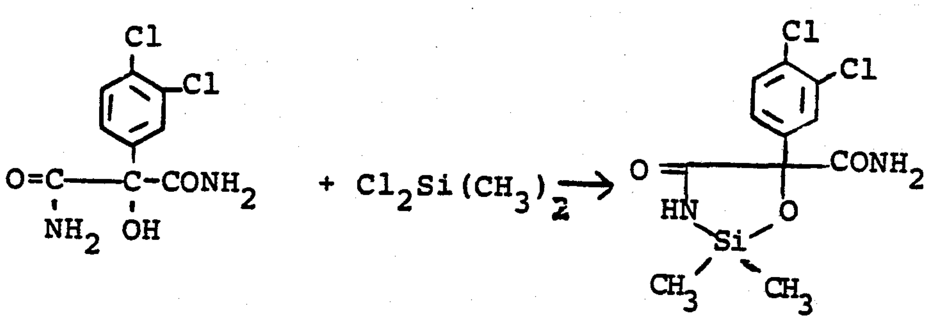

Verbindungen der Formel II sind zum Teil bekannt z.B. aus DE-OS 3 140 275, oder können nach bekannten Methoden hergestellt werden.

Verbindungen der Formel II sind zum Teil Gegenstand einer älteren noch nicht zum Stand der Technik gehörenden Anmeldung der Anmelderin Deutsche Anmeldung P 33 08462.9. Sie können nach den darin angegebenen Verfahren hergestellt werden.

Im einzelnen seien folgende Verbindungen der Formel II genannt:

Hydroxymalonsäurediamide wie Phenyl-hydroxy-malonsäurediamid, 2-, 3-, 4-Chlorphenylhydroxy-malonsäurediamid, 2,3-Dichlorphenyl-hydroxy-malonsäurediamid, 3,4-Dichlorphenyl-hydroxy-malonsäurediamid, 3,5-Dichlor phenyl-hydroxy-malonsäurediamid, 2,4-Dichlor phenyl-hydroxy-malonsäurediamid, 2,5-Dichlor phenyl-hydroxy-malonsäurediamid, 2,6-Dichlor phenyl-hydroxy-malonsäurediamid, 2-, 3-, 4-Nitrophenyl-hydroxy-malonsäurediamid, 2-Chlor - methylphenyl-hydroxy-malonsäurediamid, 2-, 3-, 4-Trifluormethylphenyl-hydroxy-malonsäurediamid, 2-, 3-, 4-Methoxyphenyl-hydroxy-malonsäurediamid, 2,6-Dimethoxyphenyl-hydroxy-malonsäurediamid, 2-, 3-, 4-Tolyl-hydroxy-malonsäurediamid, 2-, 3-, 4-Trifluormethoxyphenylhydroxy-malonsäurediamid, 2-, 3-, und 4-Fluorphenyl-hydroxymalonsäurediamid, 3,4-Dichlorphenyl-hydroxy-malonsäureamid-methylamid, Phenyl-hydroxy-malonsäure-diethylamid-amid, Cyclohexylhydroxy-malonsäure-methyl-

ester-amid, 4-Amino-, 4-Methoxy-, 4-Hydroxy-, 3,5-dichlorphenyl-hydroxy-malonsäure-diamid, 3,4-Dichlorphenylhydroxy-malonsäure-dimethylester, 3-Chlorphenyl-hydroxy-malonsäure-diethylester, 3,4,5-Trichlorphenyl-hydroxymalonsäuremethylester-amid, 4-Chlorphenyl-hydroxy-malonsäure-isopropylesteramid, 3,5-Dichlorphenyl-hydroxy-malonsäure-amidmorpholinylamid, 3,4-Dichlorphenyl-hydroxy-malonsäure-bis-isopentylamid, Phenylhydroxy-malonsäure-bismethylamid, Cyclohexyl-hydroxy-malonsäurediamid, 2,3,4-; 2,3,6-; 3,4,5-Trichlorphenyl-hydroxy-malonsäure-diamid, 2,3,4,5-; 2,3,5,6-Tetrachlorphenyl-hydroxy-malonsäurediamid, Pentachlorphenyl-hydroxy-malonsäurediamid.

α, α'-Disubstituierte Hydroxyessigsäureamide wie α-Trichlormethyl-α'-phenyl, α-t-Butyl-α'-4-chlorphenoxymethyl, α-t-Butyl-α'-2,4-dichlorphenoxymethyl, α-t-Butyl-α'-4-fluorphenoxymethyl, α-t-Butyl-α'-2,6-di-chlorphenoxymethyl, α t-Butyl-α'-phenylsulfonylmethyl, α-t-Butyl-α'-chlormethyl, α-t-Butyl-α'-3,4-dichlorphenoxymethyl, α-Phenyl-α'-ethoxycarbonylmethyl, α-t-Butyl-α'-4-fluor-2-chlor-phenoxymethyl, α-t-Butyl-α'-4-fluor-2-brom-phenoxymethyl, α-t-Butyl-α'-phenoxymethyl-hydroxyessigsäureamid.

Die Verbindungen der Formel III sind bekannt. Es seien genannt: Thionylchlorid, Schwefeldichlorid, Di($C_{1-4}$-alkyl)-silyldichlorid.

Besonders bevorzugt wird die Umsetzung mit Thionylchlorid durchgeführt.

Wird als Verbindung der Formel III Thionylchlorid eingesetzt, so wird die Reaktion mit den Verbindungen der Formel II vorzugsweise ohne Verwendung von Verdünnungsmitteln in einem großen Überschuß an Thionylchlorid durchgeführt. Dabei werden die Verbindungen der Formel II entweder zu der vorgelegten Menge Thionylchlorid zugegeben oder das Thionylchlorid wird zu der Verbindung der Formel II zugegeben. Thionylchlorid dient bei dieser Arbeitsweise als Reaktant und als Verdünnungsmittel. Die Reaktion wird bei erhöhter Temperatur im Bereich von 50 - 150°C bevorzugt 60 - 100°C besonders bevorzgut beim Siedepunkt des Gemisches unter Rückflußkühlung durchgeführt.

Die Reaktion wird bevorzugt bei Normaldruck durchgeführt. Das Arbeiten unter Überdruck ist möglich.

Die Aufarbeitung erfolgt durch Abdestillieren des Thionylchlorid, Nachwaschen des Rückstandes z.B. mit Ether und Trocknen des Rückstandes.

U.U. kann die Verbindung der Formel I auch direkt vom überschüssigen Thionylchlorid abfiltriert, gewaschen und getrocknet werden.

Die Umsetzung der Verbindungen der Formel II mit Thionylchlorid oder Schwefeldichlorid, oder Dialkylsilyldichlorid kann in Gegenwart von Verdünnungsmittels erfolgen.

Als Verdünnungsmittel werden bevorzugt eingesetzt gegebenenfalls chlorierte Kohlenwasserstoffe wie Benzol, Toluol, Xylol, Cyclohexan, Petrolether, Methylenchlorid, Chloroform, Tetrachlorkohlenstoff, Chlorbenzol, Ether wie Diethylether, Dimethylether, Dioxan, Tetrahydrofuran, Ester wie Essigester.

Die Verbindungen der Formel II werden im Verdünnungsmittel gelöst oder suspendiert und mit Thionylchlorid oder Schwefeldichlorid oder Dialkylsilyldichlorid versetzt.

Die Verbindungen der Formel II und III können in äquimolaren Verhältnis eingesetzt werden. Bevorzugt ist jedoch ein 1-2-facher Überschuß an Verbindung der Formel III wie z.B. Thionylchlorid.

Die Umsetzung erfolgt bei Temperaturen von 40 - 150°C bevorzugt bei 40 -100°C. Besonders vorteilhaft wird beim Siedepunkt des Gemisches unter Rückflußkühlung gearbeitet.

Es wird unter Normaldruck gearbeitet. Es kann jedoch auch unter Überdruck gearbeitet werden. Die Aufarbeitung erfolgt nach Abdestillieren von Thionylchlorid bzw. Schwefeldichlorid oder Dialkylsilyldichlorid und Verdünnungsmittel wie weiter oben beschrieben z.B. durch Umkristallisieren und Trocknen des Rückstandes.

Die nach Verfahren 2a erhältlichen Verbindungen der Formel I in welcher $R^2$ für Wasserstoff steht können gegebenenfalls in ihre Salze überführt werden indem man sie mit der äquimolaren Menge einer Base behandelt. Als solche kommen in frage Alkali- oder Erdalkalihydroxide wie NaOH, $Ca(OH)_2$, KOH, Alkali- oder Erdalkalialkohlate wie Natriummethylat oder -ethylat, tertiäre Amine wie Trimethylamin, Triethylamin, Pyridin oder Morpholin.

Verfahren 2b:

Die Umsetzung der Verbindungen der Formel IV mit Verbindungen der Formel V erfolgt gegebenenfalls in Gegenwart von Verdünnungsmitteln und Katalysatoren. Sie läßt sich z.B. durch das folgende Formelschema beschreiben:

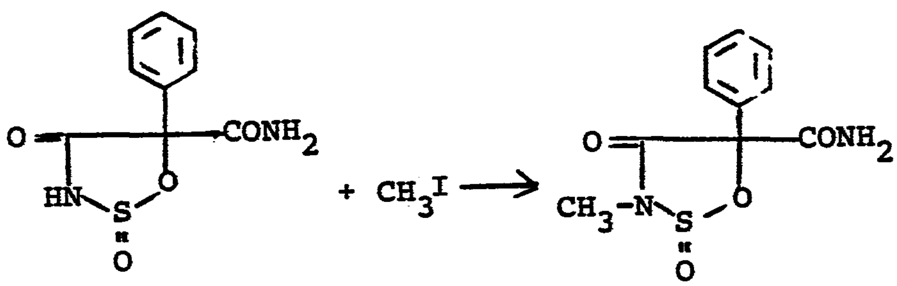

Die Verbindungen der Formel IV sind nach den oben zur Herstellung der Verbindungen der Formel I beschriebenen Verfahren erhältlich. Bevorzugt werden die oben als bevorzugt genannten Verbindungen der Formel I in welcher $R^3$ für Wasserstoff steht eingesetzt.

Die Verbindungen der Formel V sind bekannt. Als Beispiele für Verbindungen der Formel (V) seien genannt:

Alkylierungsmittel wie Methylbromid, Methyliodid, Methylchlorid, Ethylbromid, Ethylchlorid, Ethyliodid, 1-Iodpropan, 2-Iodpropan, Allylchlorid, Allylbromid, Propargylbromid, Propargylchlorid, Benzylchlorid, Benzylbromid, 3,4-Dichlorbenzylchlorid, Dimethylsulfat, Diethylsulfat.

Acylierungsmittel wie Benzoylchlorid, 3,4-Dichlorbenzoylchlorid, 3-Chlorbenzoylbromid, Acetylchlorid, Acetanhydrid, Propionylchlorid, Benzoylcyanid, Chlorameisensäueethylester, Chlorameisensäuremethylester, Methylsulfamoylchlorid, Dimethylcarbamidsäurechlorid, Chlorameisensäurephenylester.

Isocyanate wie Methylisocyanat, Ethylisocyanat, Propylisocyanat, Butylisocyanat, Dodecylisocyanat, Phenylisocyanat, p-Chlorbenzoylisocyanat, o-Chlorbenzoylisocyanat, Sulfonylisocyanate wie Benzolsulfonylisocyanat, 3,4-Dichlorbenzoylisocyanat.

Sulfenylierungsmittel wie Trihalogenmethylsulfensäurechlorid, 2,3-Trichlormethyl-, Dichlorfluormethyl-, Trifluormethylsulfensäurechlorid, gegebenenfalls substituiertes Phenylsulfensäurechlorid wie z.B. Phenyl-, p-Chlorphenyl-, p-Trifluormethylphenyl-, 3,4-Dichlorphenylsulfensäurechlorid. Silylierungsmittel wie z.B. Trimethylsilylcyanid oder -chlorid. Sulfonylierungsmittel wie z.B. Tosylchlorid, Phenylsulfonylchlorid, Chlorphenylsulfonylchlorid.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibtuylether, Glykoldimethylether und Diglykoldimethyleter, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Säureakzeptoren können alle üblichen Säurebindemittel verwendet werden. Hierzu gehören vorzugsweise Alkalicarbonate, -hydroxide oder -alkoholate, wie Natrium- oder Kaliumcarbonat, Natrium- und Kaliumhydroxid, Natrium- und Kaliummethylat bzw. -ethylat, ferner aliphatische, aromatische oder heterocyclische Amine, beispielsweise Trimethylamin, Triethylamin, Tributylamin, Dimethylanilin, Dimethylbenzylamin und Pyridin.

Als Katalysatoren können Verbindungen verwendet werden, welche gewöhnlich bei Reaktionen in Zweiphasensystemen

aus Wasser und mit Wasser nicht mischbaren organischen Lösungsmitteln zum Phasentransfer von Reaktanden dienen (Phasentransferkatalysatoren). Als solche sind vor allem Tetraalkyl- und Trialkylaralkyl-ammoniumsalze mit vorzugsweise 1 bis 10, insbesondere 1 bis 8 Kohlenstoffen je Alkylgruppe, vorzugsweise Phenyl als Arylbestandteil der Aralkylgruppe und vorzugsweise 1 bis 4, insbesondere 1 oder 2 Kohlenstoffatomen im Alkylteil der Aralkylgruppen bevorzugt. Hierbei kommen vor allem die Halogenide, wie Chloride, Bromide und Iodide, vorzugsweise die Chloride und Bromide infrage. Beispielhaft seien Tetrabutylammoniumbromid, Benzyl-triethylammoniumchlorid und Methyltrioctylammoniumchlorid genannt.

Die Reaktionstemperatur wird zwischen etwa 0°C und 130°C, vorzugsweise zwischen etwa 20°C und 60°C gehalten. Das Verfahren wird vorzugsweise bei Normaldruck durchgeführt.

Die Aufarbeitung erfolgt in üblicher Weise.

Verfahren 2c:

Die Umsetzung der Verbindungen der Formel II mit Silyldicyaniden der Formel IX erfolgt wie bei Verfahren 2a (Variante unter Verwendung von Verdünnungsmitteln) beschrieben.

Bevorzugt wird das Verfahren in Gegenwart katalytischer (0,01 - 5 Gew.-%) bis äquimolarer Mengen einer Base durchgeführt. Als Basen seien bevorzugt tert. Amine wie Trimethylamin, Triethylamin oder Pyridin genannt.

Verfahren 2d:

Die Umsetzung der Verbindungen der Formel X mit Verbindungen der Formel XI kann durch folgendes Formelschema wiedergegeben werden:

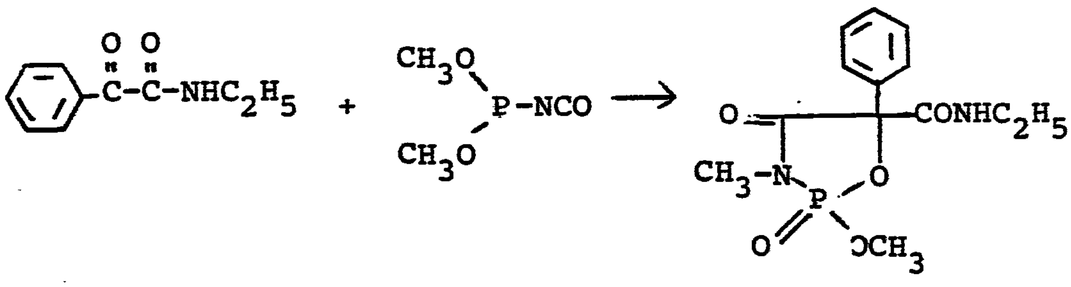

Verbindungen der Formel X sind bekannt oder können analog zu bekannten Verfahren hergestellt werden.

Verbindungen der Formel XI sind bekannt oder können analog zu bekannten Verfahren hergestellt werden (vgl. Journ. Gen. Chem. USSR Band 38 III (1968) S. 1479 - 1482).

Die Umsetzung erfolgt in einem gegenüber den Edukten inerten Verdünnungsmittel. Als solche kommen die bei Verfahren 2a erwähnten infrage.

Die Verbindungen der Formeln X und XI werden bevorzugt in äquimolarer Menge eingesetzt.

Die Reaktion wird bei Temperaturen von -20°C bis +50°C bevorzugt -10°C bis +20°C durchgeführt.

Die Edukte werden gemischt und gerührt, bis die Umsetzung vollständig ist. Das Ende der Reaktion kann spektroskopisch durch Verschwinden der NCO Bande in Reaktionsgemisch festgestellt werden.

Die Aufarbeitung erfolgt nach Abdestillieren des Verdünnungsmittels in üblicher Weise.

Wie bereits erwähnt ist die Herstellung der Verbindungen der Formel II Gegenstand einer älteren nicht vorveröffentlichten Anmeldung der Anmelderin Deutsche Anmeldung P 33 0462.9.

Im Folgenden wird ihre Herstellung in allgemeiner Form beschrieben:

3. Verbindungen der Formel XII

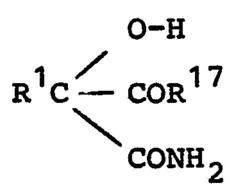

XII

in welcher

$R^1$ die unter 1 angegebene Bedeutung hat und

$R^{17}$ für Amino, gegebenenfalls substituiertes Alkyloxy oder Cycloalkyloxy, ferner für Alkylamino, Arylamino, Dialkylamino, Cycloalkylamino, Alkenylamino, stickstoffhaltige gesättigte heterocyclische Reste die gegebenenfalls weitere Heteroatome enthalten, wobei die Reste gegebenenfalls substituiert sein können, steht

erhält man indem man Verbindungen der Formel XIII

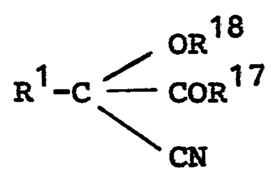

XIII

in welcher

$R^1$ die unter 1 angegebene Bedeutung hat

$R^{18}$ für Wasserstoff oder $Si(CH_3)_3$ steht

$R^{17}$ die oben angegebene Bedeutung hat

mit anorganischen Säuren umsetzt.

4. Verbindungen der Formel XIV

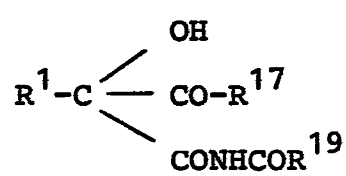

XIV

in welcher

$R^1$ die unter 1 angegebene Bedeutung hat

$R^{17}$ die unter 3 angegebene Bedeutung hat und

$R^{19}$ für Wasserstoff, gegebenenfalls substituiertes Alkyl, Alkenyl, Cycloalkenyl oder Aryl steht

erhält man,

indem man Verbindungen der Formel XV

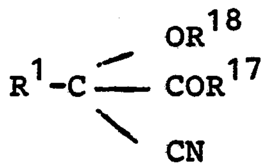

XV

in welcher

$R^1$, $R^{17}$ die oben angegebene Bedeutung haben und

$R^{18}$ für Wasserstoff oder $Si(CH_3)_3$ steht,

mit Säuren der Formel XVI

$$R^{19}\text{-COOH} \qquad XVI$$

in welcher

$R^{19}$ die oben angegebene Bedeutung hat

in Gegenwart von anorganischen Säuren

umsetzt.

5. Verbindungen der Formel XVII

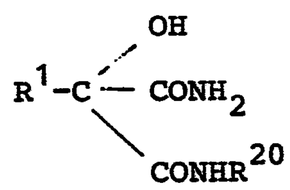

XVII

in welcher

$R^1$ die unter 1 angegebene Bedeutung hat,

$R^{20}$ für Alkyl, Cycloalkyl, Alkenyl, Amino, Alkylamino, Arylamino, Acylamino, Dialkylamino, Alkylarylamino

erhält man, indem man Verbindungen der Formel XVIII

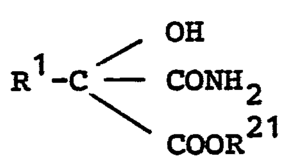

XVIII

in welcher

$R^1$ die oben angegebene Bedeutung hat und

$R^{21}$ für Alkyl oder Cycloalkyl steht,

mit Verbindungen der Formel XIX

$R^{20}NH_2$ XIX

in welcher

$R^{20}$ die oben angegebene Bedeutung hat

umsetzt.

6. Verbindungen der Formel XX

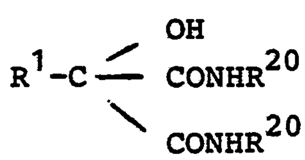

XX

in welcher

$R^1$ und $R^{20}$ die unter 5 angegebene Bedeutung haben

erhält man

indem man Verbindungen der Formel XXI

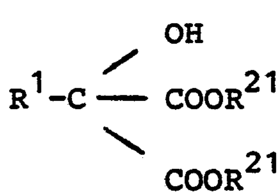

XXI

in welcher

$R^1$ die oben angegebene Bedeutung hat und

$R^{21}$ gleich oder verschieden sind und für Alkyl oder Cycloalkyl stehen,

mit Verbindungen der Formel XIX

$$R^{20}NH_2 \qquad XIX$$

in welcher

$R^{20}$ die oben angegebene Bedeutung hat,

umsetzt.

7. Verbindungen der Formel XXII

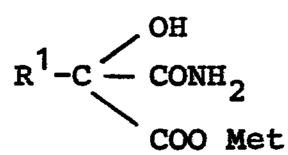

XXII

in welcher

$R^1$ die unter 1. angegebene Bedeutung hat,

Met für ein Äquivalent eines Alkali- oder Erdalkalikations steht,

erhält man

indem man Verbindungen der Formel XVIII

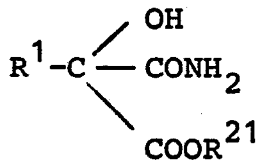

XVIII

in welcher

$R^1$, $R^{21}$ die unter 5. angegebene Bedeutung haben

mit Verbindungen der Formel XXIII

Met-OH XXIII

in welcher

Met die oben angegebene Bedeutung hat

umsetzt.

8. Verbindungen der Formel XXIV

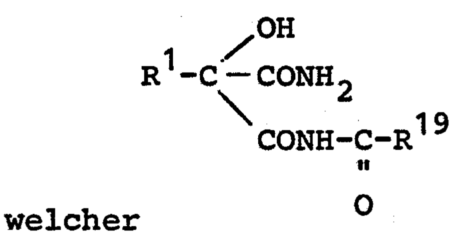

XXIV

in welcher

$R^1$ die unter 1 angegebene Bedeutung hat und

$R^{19}$ die unter 4 angegebene Bedeutung hat,

erhält man

indem man Verbindungen der Formel XXV

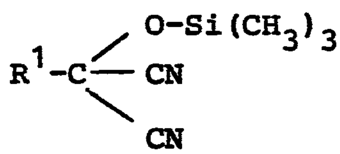

XXV

in welcher

$R^{1}$ die oben angegebene Bedeutung hat,

mit Säuren der Formel XVI

$$R^{19}\text{-COOH} \qquad \text{XVI}$$

in welcher

$R^{19}$ die oben angegebene Bedeutung hat

oder ihren Anhydriden in Gegenwart von anorganischen Mineralsäuren umsetzt.

9. Verbindungen der Formel XXVI

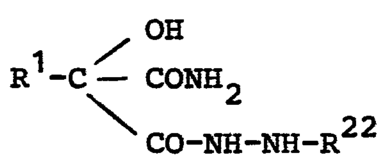

XXVI

in welcher

$R^1$ die unter 1 angegebene Bedeutung hat

$R^{22}$ für Alkylaminocarbonyl, Alkylaminothiocarbonyl, Arylaminocarbonyl, Arylaminothiocarbonyl, Alkylsulfonylaminocarbonyl, Arylsulfonylaminocarbonyl, Alkylcarbonylaminocarbonyl, Arylcarbonylaminocarbonyl sowie die entsprechenden Thiocarbonyle, steht,

erhält man

indem man Verbindungen der Formel XXVII

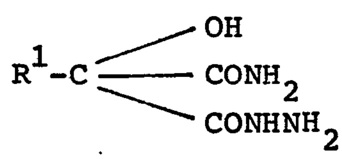

XXVII

in welcher

$R^1$ die oben angegebene Bedeutung besitzt

mit Iso-(thio)-cyanaten der Formel XXVIII

$$R^{23}NCO(S) \qquad XXVIII$$

in welcher

$R^{23}$ für Alkyl, Aryl, Alkylsulfonyl, Arylsulfonyl, Alkylcarbonyl, Arylcarbonyl steht

umsetzt.

10. Verbindungen der Formel XXIX

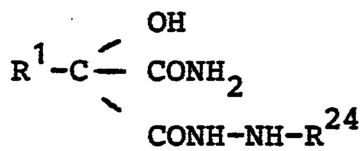

XXIX

in welcher

$R^1$ die unter 1 angegebene Bedeutung hat

$R^{24}$ für Alkylcarbonyl, Arylcarbonyl, Alkylsulfonyl, Arylsulfonyl steht

erhält man

indem man Verbindungen der Formel XXVII

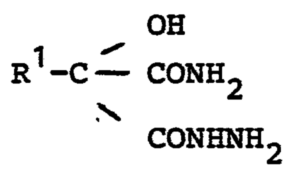

XXVII

in welcher

$R^1$ die oben angegebene Bedeutung hat

mit Acylierungsmitteln der Formel XXX

$R^{24}$-A XXX

in welcher

$R^{24}$ die oben angegebene Bedeutung hat

A für Halogen, CN, $C_{1-4}$-Alkylcarbonyloxy, Arylcarbonyloxy steht

umsetzt.

11. Verbindungen der Formel XXXI

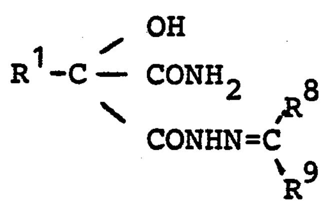

XXXI

in welcher

$R^1$, $R^8$, $R^9$ die unter 1 angegebene Bedeutung besitzen

erhält man

indem man Verbindungen der Formel XXVII

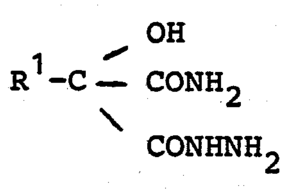

XXVII

in welcher

$R^1$ die oben angegebene Bedeutung besitzt

mit Carbonylverbindungen der Formel XXXII

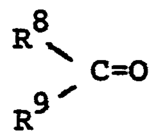

XXXII

in welcher

$R^8$ und $R^9$ die oben angegebene Bedeutung besitzen

umsetzt.

12. Verbindungen der Formel XXXIII

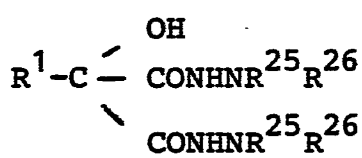

XXXIII

in welcher

$R^1$ die unter 1 angegebene Bedeutung hat

$R^{25}$ für Wasserstoff steht,

$R^{26}$ für die bei $R^{23}$ und $R^{24}$ unter Verfahren 9 und 10 (oben) angegebenen Bedeutungen steht oder

$R^{25}$ und $R^{26}$ gemeinsam für den Rest 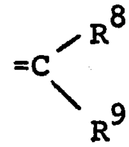

wobei $R^8$ und $R^9$ die unter 1 angegebene Bedeutung hat stehen,

erhält man,

indem man die Verbindungen der Formel XXXIV

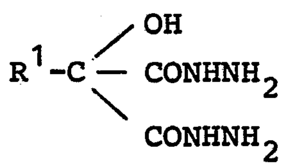

XXXIV

mit Verbindungen der Formeln XXXIII, XXXV oder XXXVII

$R^{23}$ NCO(S) XXVIII; $R^{24}$-A XXX; 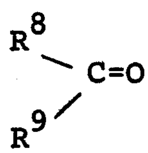 XXXII

in welchen

A, $R^{23}$, $R^{24}$, $R^8$, $R^9$ die unter 9, 10, 11 angegebenen Bedeutungen besitzen

umsetzt.

13. Die Verbindungen der Formel XII

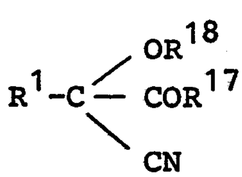

XII

in welcher

$R^1$, $R^{18}$, $R^{17}$ die unter 3 und 4 angegebene Bedeutung haben,

erhält man

indem man Verbindungen der Formel XXXV

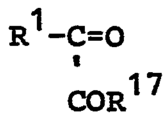 XXXV

in welcher

$R^1$ und $R^{17}$ die oben angegebene Bedeutung haben

mit HCN oder HCN-abspaltenden Verbindungen oder mit Trimethylsilylcyanid umsetzt.

14. Die Verbindungen der Formel XXI

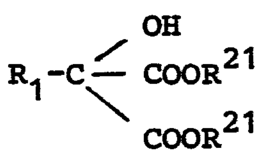 XXI

in welcher

$R^1$, $R^{21}$ die unter 6 und 8 angegebene Bedeutung haben

erhält man

indem man Verbindungen der Formel XXV

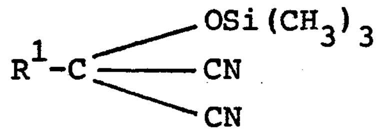

XXV

in welcher

$R^1$ die unter 1 angegebene Bedeutung hat

mit Alkoholen der Formel XXXVI

$R^{21}$-OH XXXVI

in welcher

$R^{21}$ die oben angegebene Bedeutung hat

in Gegenwart anorganischer Mineralsäuren umsetzt.

15. Verbindungen der Formel XXXVII

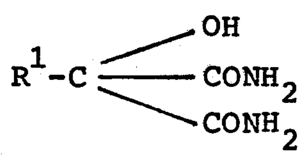

XXXVII

in welcher

$R^1$ die unter 1 angegebene Bedeutung hat

erhält man, indem man Verbindungen der Formel XXV

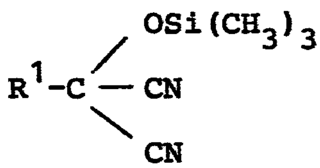

XXV

in welcher

$R^1$ die oben angegebene Bedeutung hat

mit anorganischen Säuren verseift.

Verbindungen der Formel XXV

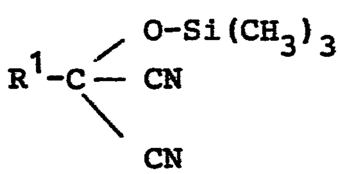

XXV

in welcher

$R^1$ die unter 1 angegebene Bedeutung hat

sind bekannt bzw. lassen sich nach bekannten Verfahren herstellen (Chem. Ber. <u>106</u>, S. 87 (1977);

Tetrahedron Letters No. 17, 1449 - 1450 (1973); DE-OS 3 140 632).

Verbindungen der Formel XXXV

$$\begin{array}{l} R^{1}-C=O \\ \quad\;| \\ \quad COR^{17} \end{array} \qquad XXXV$$

in welcher

$R^1$ und $R^{17}$ die unter 13 angegebene Bedeutung haben

sind zum Teil bekannt. Sie werden in an sich bekannter Weise hergestellt (Tetrahedron Letters 1980 S. 3539; Annalen (10) g S. 241; DE-OS 2 249 820, DE-OS 2 708 189).

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der

Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form

beispielsweise des Tauchens (Dippen), Sprühens (Sprayen), Aufgießens (pour-on and spot-on) und des Einpuderns sowie durch parenterale Anwendung in Form beispielsweise der Injektion.

Beispiel 1

144,5 g (0,55 Mol) 3,4-Dichlorphenyl-hydroxy-malonsäurediamid werden in 350 ml Thionylchlorid suspendiert. Man erhitzt das Gemisch unter Rühren 3 Stunden zum Sieden, kühlt auf 10°C und filtriert die ausgeschiedenen Kristalle ab. Die Kristalle werden mit Diethylether gewaschen und bei 50°C getrocknet.

Man erhält so 120,5 g (70,9 % der Theorie) an 5-(3,4-Dichlorphenyl)-5-aminocarbonyl-1,2,3-oxathiazolidin-4-on-2-oxid in Form schwach gelblicher Kristalle, die zwischen 191 und 192°C unter Zersetzung schmelzen.

Beispiel 2

Zu einer frisch bereiteten Lösung von 7 g Natriummethylat in 200 ml wasserfreiem Methanol werden 40,2 g (0,13 Mol) 5-(3,4-Dichlorphenyl)-5-aminocarbonyl-1,2,3-oxathiadiazolidin-4-on-2-oxid eingetragen. Nach einstündigem Rühren bei Raumtemperatur wird das Lösungsmittel unter vermindertem Druck abdestilliert, der verbleibende feste Rückstand mit Diethylether verrieben und bei Raumtemperatur getrocknet.

Man erhält so 43 g (100 % der Theorie) des Natrium-Salzes von 5-(3,4-Dichlorphenyl)-5-aminocarbonyl-1,2,3-oxathiazolidin-4-on-2-oxid, das sich oberhalb 250°C unter Dunkelfärbung langsam zersetzt.

Beispiel 3

Zu einer Lösung von 6,2 g (0,02 Mol) 5-(3,4-Dichlorphenyl)-5-aminocarbonyl-1,2,3-oxathiazolidin-4-on-2-oxid und 2,02 g (0,02 Mol) Triethylamin in 50 ml Acetonitril werden 31,2 g (0,2 Mol) Ethyljodid zugegeben. Die Lösung wird vier Stunden zum Sieden erhitzt und anschließend unter vermindertem Druck eingedampft. Der Rückstand wird in 50 ml Dichlormethan aufgenommen und dreimal mit je 20 ml Wasser gewaschen. Die organische Phase eingedampft und der Rückstand mit Diethylether verrieben. Es werden so 4,7 g (70 % der Theorie) an 3-Ethyl-5-(3,4-dichlorphenyl)-5-aminocarbonyl-1,2,3-oxathiazolidin-4-on-2-oxid vom Schmelzpunkt 127-128°C erhalten.

Beispiel 4

Zu einer Suspension von 38 g (0,14 Mol) (4-Chlorphenoxymethyl)-α'-tert.-butyl-hydroxy-acetamid in 140 ml Dichlormethan werden 25 g (0,21 Mol) Thionylchlorid zugegeben. Das Gemisch wird unter Rühren zwölf Stunden zum Sieden erhitzt. Anschließend wird die Lösung zur Trockne eingedampft.

Man erhält so 44,4 g (100 % der Theorie) an 5-(4-Chlorphenoxymethyl)-5-tert.-butyl-1,2,3-oxathiazolidin-4-on-2-oxid vom Schmelzpunkt 132-134°C. Nach Umlösen aus Diethylether/Petrolether steigt der Schmelzpunkt auf 136-137°C an.

Analog Beispielen 1 - 4 werden die folgenden 1,2,3-Oxathiazolidin-4-on-2-oxide erhalten:

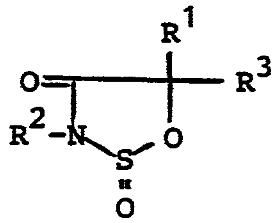

| Beispiel Nr. | $R^1$ | $R^2$ | $R^3$ | physikalische Eigenschaften |
|---|---|---|---|---|
| 5 | t-Butyl | H | $CH_2Cl$ | Fp. 84 - 88°C |
| 6 | t-Butyl | H | 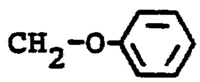 | |
| 7 | t-Butyl | H | 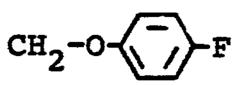 | Fp. 91 - 93°C |
| 8 | t-Butyl | H | 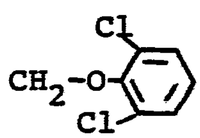 | Fp. 198 - 199°C |
| 9 | t-Butyl | H | 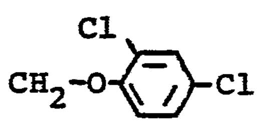 | Fp. 189°C |
| 10 | t-Butyl | H | 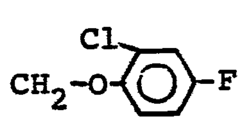 | Fp. 171°C |
| 11 | 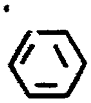 | H | $CCl_3$ | Fp. 113 - 116°C |

| Beispiel Nr. | $R^1$ | $R^2$ | $R^3$ | physikalische Eigenschaften |
|---|---|---|---|---|
| 12 | 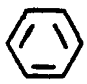 | H | $-CH_2-COOC_2H_5$ | Fp. 136 - 137°C |
| 13 | 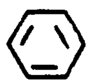 | H | 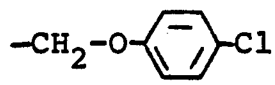 | |
| 14 | 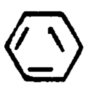 | $CH_3$ | $CONH_2$ | Fp. 173 - 173,5°C |
| 15 | 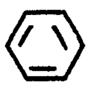 | H | $CONH_2$ | Fp. 184 - 185°C |
| 16 | 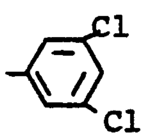 | H | $CONH_2$ | Fp. 223°C |
| 17 | 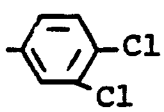 | $SCCl_2F$ | $CONH_2$ | viskoses Öl |
| 18 | 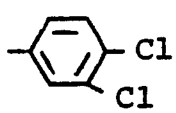 | $\overset{\oplus}{N}(C_2H_5)$ | $CONH_2$ | viskoses Öl |
| 19 | 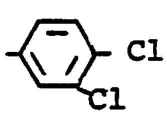 | 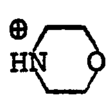 | $CONH_2$ | Fp. 174°C |
| 20 | 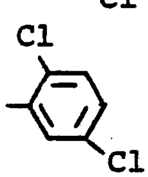 | $C_2H_5$ | $CONH_2$ | Fp. 135°C Zers. |

| Beispiel Nr. | $R^1$ | $R^2$ | $R^3$ | physikalische Eigenschaften |
|---|---|---|---|---|
| 20 a | 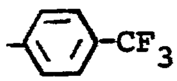 | $CH_3$ | $CONH_2$ | Fp. 174°C |
| 20 b | " | $C_2H_5$ | $CONH_2$ | Fp. 136°C |
| 20 c | 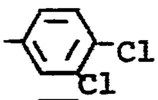 | $-CH_2-CH=CH_2$ | $CONH_2$ | Fp. 116-117°C |
| 20 d | 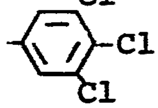 | $-CH(CH_3)_2$ | $CONH_2$ | Fp. 136-138°C |
| 21 | 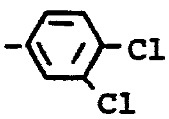 | $CH_3$ | $CONH_2$ | Fp. 161-163°C |
| 22 | 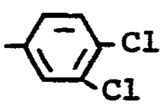 | $-CH_2-C\equiv CH$ | $CONH_2$ | Fp. 167 - 168°C |
| 23 | 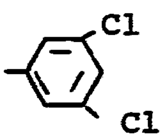 | $Na^{\oplus}$ | $CONH_2$ | Fp. 260°C |
| 24 | 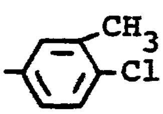 | H | $CONH_2$ | Fp. 198°C |
| 25 | 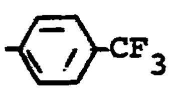 | H | $CONH_2$ | Fp. 178 - 179°C |
| 26 | 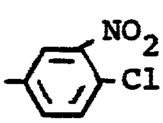 | H | $CONH_2$ | Fp. 184 - 185°C |
| 27 | 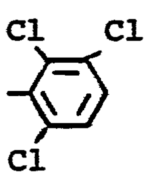 | H | $CONH_2$ | Fp. 189°C |

| Beispiel Nr. | $R^1$ | $R^2$ | $R^3$ | physikalische Eigenschaften |
|---|---|---|---|---|
| 28 | $-CH_2-O-$ 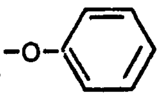 | H | $CONH_2$ | Fp. 201°C |
| 28a | 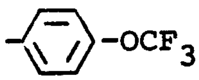 | H | $CONH_2$ | Fp. 178-179°C |
| 28b | 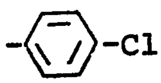 | H | $CONH_2$ | Fp. 196°C |
| 28c | 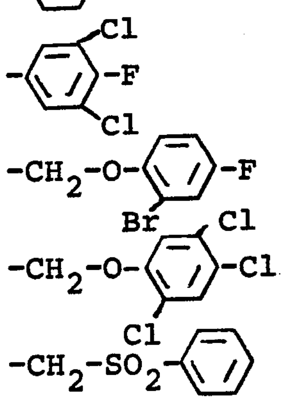 | H | $CONH_2$ | Fp. 228°C |
| 28d | $-CH_2-O-$ | H | $t-C_4H_9$ | Fp. 180-181°C |
| 28e | $-CH_2-O-$ | H | $t-C_4H_9$ | Fp. 202-203°C |
| 28f | $-CH_2-SO_2-$ | H | $t-C_4H_9$ | Fp. 208°C |
| 28g | $-CH_2O-$ 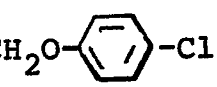 | $CH_3$ | $t-C_4H_9$ | $n_D^{20}$ 1,5330 |
| 28h | " | $C_2H_5$ | " | $n_D^{20}$ 1,5280 |
| 28i | " | $-CH_2-C{\equiv}CH$ | " | $n_D^{20}$ 1,5404 |
| 28j | $-CH_2-O-$ 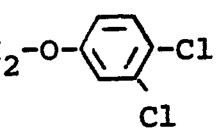 | $CH_3$ | " | Fp. 110-116°C |
| 28k | " | $C_2H_5$ | " | Fp. 101-104°C |
| 29 | " | $-CH_2-C{\equiv}CH$ | " | Fp. 102-104°C |

Beispiel 30

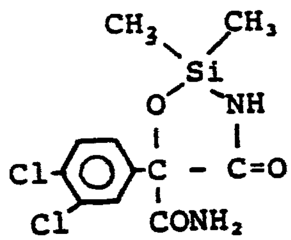

26,3 g 3,4-Dichlorphenylhydroxymalonsäurediamid wurden in 100 ml absolutem Dioxan warm gelöst und anschließend mit einer Lösung von 11 g Dimethylsilyldicyanid in 50 ml absolutem Dioxan versetzt. Es wurde 8 Stunden am Rückfluß erhitzt, wobei die Kühlwassertemperatur 30°C betrug um entstehende Blausäure abzutrennen.

Anschließend wurde eingeengt und der Rückstand mit 250 ml Toluol augekocht. Nach dem Abkühlen wurde abgesaugt. Es blieben 28 g 5-Aminocarbonyl-5-(3,4-dichlorphenyl)-2-dimethyl-1-oxa-3-aza-2-sila-cyclopentan-4-on.
Schmelzpunkt: 206 - 207°C.

Beispiel 31

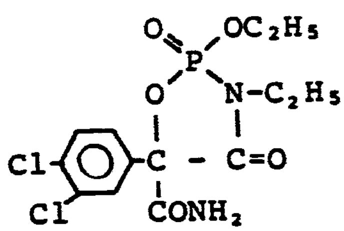

10,9 g 3,4-Dichlorphenylglyoxylsäureamid wurden in 200 ml absolutem Dimethoxyethan gelöst und bei 15°C mit einer

Lösung von 8,2 g Diethylphosphorigsäureisocyanat in 10 ml Dimethyloxyethan versetzt. Die Lösung wurde bei Raumtemperatur über Nacht stehengelassen und anschließend eingeengt. Es wurde noch zweimal mit Chloroform versetzt und eingeengt, gegen Ende im Hochvakuum bei 70°C Badtemperatur. Es bleiben 20g glasartiger Rückstand, welcher sich spektroskopisch als 2-Ethoxy-3-ethyl-4-oxo-5-(3,4-dichlorphenyl)-1,3,2-oxazaphospholidin-5-aminocarbonyl-2-oxid erwies.

Analog Beispielen 29, 30, 31 werden die folgenden Verbindungen hergestellt:

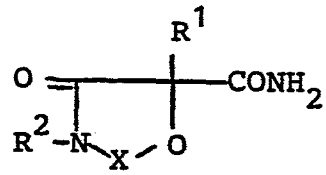

| Beispiel Nr. | $R^1$ | $R^2$ | X | Physikalische Daten |
|---|---|---|---|---|
| 32 | 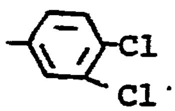 | $CH_3$ | $>Si(CH_3)_2$ | |
| 33 | 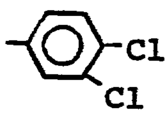 | $CH_3$ | 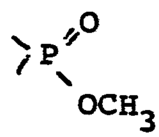 | |
| 34 | 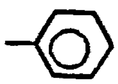 | $CH_3$ | 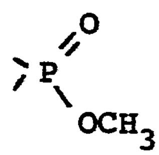 | |

Herstellung der Vorprodukte:

Beispiel a)

48,8 g (0,15 Mol) α-(4-Chlorphenoxymethyl)-α'-tert.-butyl-trimethylsilyloxy-acetonitril werden bei 15 - 20°C portionsweise in 110 ml 96 %ige Schwefelsäure eingerührt. Man hält das Gemisch noch zwei Stunden bei dieser Temperatur, gießt dann auf Eis und extrahiert mit Dichlormethan. Die organische Phase wird mit Wasser gewaschen, über wasserfreiem Natriumsulfat getrocknet und anschließend zur Trockne eingedampft. Durch Verreiben des Rückstandes mit Diethylether erhält man 20 g (49,4 % der Theorie) an α-(4-Chlorphenoxymethyl)-α'-tert.-butyl-hydroxyacetamid vom Schmelzpunkt 160°C.

Analog Beispiel a) werden folgende Verbindungen erhalten:

$a_1$ α-(3,4-Dichlorphenoxymethyl)-α'-t-butyl-hydroxyacetamid Fp. 166°C

$a_2$ α-(2-Brom-4-fluorphenoxymethyl)-α'-t-butyl-hydroxyacetamid Fp. 116-117°C

$a_3$ α-(Phenylsulfonylmethyl)-α'-t-butyl-hydroxyacetamid Fp. 187-188°C

$a_4$ α-(2,4,5-Trichlorphenoxymethyl)-α'-t-butyl-hydroxyacetamid Fp. 145-146°C

$a_5$ α-(2,4-Dichlorphenoxymethyl)-α'-t-butyl-hydroxyacetamid Fp. 122-123°C

$a_6$ α-(4-Chlorphenoxymethyl)-α'-t-butyl-hydroxyacetamid Fp. 160°C

$a_7$ α-(2,6-Dichlorphenoxymethyl)- α-t-butyl-hydroxyacetamid Fp. 169-170°C

$a_8$ α-(4-Fluorphenoxymethyl)- α-t-butyl-hydroxy-acetamid

$a_9$ α-(2-Chlor-4-fluorphenoxymethyl)- α t-butyl-hydroxyacetamid

$a_{10}$ α-(Piperidinomethyl)- α'-t-butyl-hydroxy-acetamid Fp. 106-107°C

$a_{11}$ α-(Morpholinomethyl)- α'-t-butyl-hydroxy-acetamid Fp. 103-106°C

$a_{12}$ α-(Pyrazolinomethyl)- α'-t-butyl-hydroxy-acetamid Fp. 138°C

$a_{13}$ α-(Propoxymethyl)- α'-t-butyl-hydroxyacetamid Fp. Fp. 106-107°C

$a_{14}$ α-(Methoxymethyl)- α'-t-butyl-hydroxy-acetamid Fp. 130-131°C

$a_{15}$ α-(Ethoxymethyl)- α'-t-butyl-hydroxyaetamid Fp. 115-116°C

$a_{16}$ α-(Propoxymethyl)- α'-t-butyl-hydroxyacetamid Fp. 124-125°C

$a_{17}$ α-Chlormethyl- α'-t-butyl-hydroxyacetamid Fp. 123°C

$a_{18}$ α-Chlormethyl- α'-methylcyclohexyl-hydroxy-acetamid Fp. 80-100°C

$a_{19}$ α-Brommethyl- α'-(4-Chlorphenyl)-hydroxy-acetamid Fp. 108-109°C

$a_{20}$ α-(2,4-Dichlorphenyl)- α'-chlormethyl-hy-droxyacetamid Fp. 137-138°C

$a_{21}$ α-(2,4-Dichlorphenyl)- α'-brommethyl-hy-droxyacetamid Fp. 138-139°C

Beispiel b)

Zu einer Lösung von 99,1 g (1 Mol) Trimethylsilylcyanid und 1 ml Triethylamin in 500 ml Dichlormethan werden portionsweise 226,7 g (1 Mol) 1-(4-Chlorphenoxy)-3,3-dimethylbutan-2-on eingerührt. Die Temperatur des Reaktionsgemisches steigt dabei allmählich auf 30°C an. Man läßt über Nacht bei Raumtemperatur stehen, destilliert das Lösungsmittel unter vermindertem Druck ab und reibt den verbleibenden kristallinen Rückstand mit wenig Petrolether an. Es werden so 280,9 g (86,2 % der Theorie) α-(4-Chlorphenoxymethyl)-α'-tert.-butyl-trimethylsilyloxy-acetonitril vom Schmelzpunkt 98°C erhalten.

Analog Beispiel b) werden folgende Verbindungen erhalten:

$b_1$ α-(2,4-Dichlorphenoxymethyl)-α'-t-butyl-trimethylsilyloxy-acetonitril Fp. 94-95°C

$b_2$ α-Phenoxymethyl-α'-t-butyl-trimethylsilyloxy-acetonitril Fp. 61-62°C

$b_3$ α-(4-Chlorphenoxymethyl)-α'-t-butyl-trimethylsilyloxy-acetonitril Fp. 98°C

$b_4$ α-(3,4-Dichlorphenoxymethyl)-α-t-butyl-trimethylsilyloxy-acetonitril Fp. 90-91°C

$b_5$ α-(2,6-Dichlorphenoxymethyl)-α-t-butyl-trimethylsilyloxy-acetonitril Fp. 64-65°C

$b_6$ α-(4-Fluorphenoxymethyl)-α-t-butyl-trimethylsilyloxy-acetonitril Fp. 69°C

$b_7$ α-(3-Chlorphenoxymethyl)-α-t-butyl-trimethylsilyloxy-acetonitril Fp. 109-110°C

$b_8$ α-(2,4,5-Trichlorphenoxymethyl)-α-t-butyl-trimethylsilyloxy-acetonitril Fp. 69-70°C

$b_9$ α-(2-Chlor-4-fluorphenoxymethyl)-α-t-butyl-trimethylsilyloxy-acetonitril Fp. 68°C

$b_{10}$ α-(2-Brom-4-fluorphenoxymethyl)-α'-t-butyl-trimethylsilyloxy-acetonitril Fp. 47°C

$b_{11}$ α-Phenylsulfonylmethyl-α'-t-butyl-trimethylsilyloxy-acetonitril Fp. 121-122°C

Beispiel c

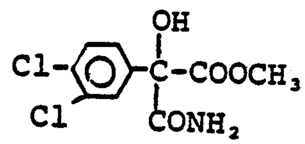

in 400 g Schwefelsäure (96 %ig) wurde bei max. 40°C 210 g 3,4-Dichlorphenyl-trimethylsilyloxy-malonsäureethylesternitril eingetropft. Es wurde noch 1 Stunde bei Raumtemperatur nachgerührt und dann in Eiswasser eingerührt.

Der zuerst schmierige, dann kristallisierende Niederschlag wurde in Essigsäureethylester aufgenommen, gewaschen, getrocknet und eingeengt.

Durch Umkristallisation aus Isopropanol wurde 3,4-Dichlorphenyl-hydroxy-malonsäuremethylesteramid vom Schmelzpunkt 132 - 133°C erhalten.

Beispiel d

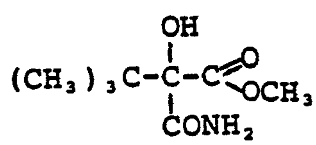

Analog zu obigem Beispiel wurden aus 187 g tert.-Butyl-trimethylsilyloxy-malonsäuremethylesternitril durch Verseifung im 500 g $H_2SO_4$ 55 g tert.-Butylhydroxymalonsäuremethylesteramid hergestellt.
Schmelzpunkt 102 - 103°C (aus Waschbenzin).

Beispiel e

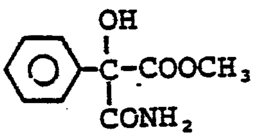

95 g Phenyl-hydroxy-malonsäuremethylesternitril werden bei 30°C zu 500 g $H_2SO_4$ getropft.

Nachdem 30 Minuten bei Raumtemperatur nachgerührt wurde, wurde in Eiswasser eingerührt, mit Methylenchlorid aufgenommen, gewaschen und eingeengt. Der Rückstand wurde aus Toluol umkristallisiert. Es wurden 72 g Phenyl-hydroxy-malonsäuremethylesteramid vom Schmelzpunkt 123 - 4°C erhalten.

Beispiel f

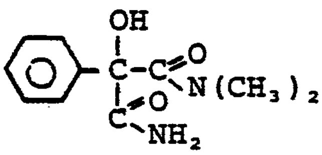

Zu 250 ml $H_2SO_4$ konz. wurden bei 40°C 63 g Phenyltrimethylsilyloxymalonsäuredimethylamidnitril getropft. Nachdem unter Rühren die Temperatur 9 h auf 40°C gehalten worden war, wurde auf Eis gegossen, mit $CH_2Cl_2$ extrahiert und die organische Phase aufgearbeitet. Es blieben 31 g Rückstand, der aus i-Propanol umkristallisiert wurde. Es bleiben 26,2 g Phenylhydroxymalonsäureamiddimethylamid; Fp. 137 -8°C.

Beispiel g

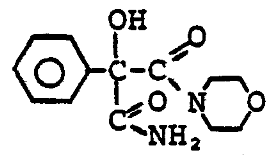

In 300 ml $H_2SO_4$ konz. wurde bei max. 40°C 90 g Phenyltrimethylsilyloxymalonsäuremorpholidnitril eingetragen. Nach 2,5 Stunden bei 40°C wurde in Eiswasser eingerührt und 3x mit Methylenchlorid extrahiert. Die organischen Phasen wurden vereinigt, mit wenig Wasser gewaschen und das Lösungsmittel abdestilliert, wobei gegen Ende Hochvakuum angelegt wurde. Es bleiben 57 g glasiges Produkt, welches spektroskopisch als Phenylhydroxymalonsäuremorpholidamid identifiziert wurde.

Beispiel h

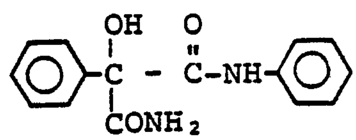

10 g Phenyltrimethylsilyloxymalonsäureanilidnitril wurden bei 30°C in 100 ml konzentrierte Salzsäure eingetragen. Die Suspension wurde anschließend 2 Stunden auf 50°C erhitzt, der Rückstand in Methylenchlorid aufgenommen, gewaschen und aufgearbeitet. Es blieben 5 g glasiges Rohprodukt, welches aus Toluol umkristallisiert wurde. Es konnten 4 g Phenylhydroxymalonsäureanilidamid isoliert werden. Fp. ab 110°C unter Zersetzung.

Beispiel i

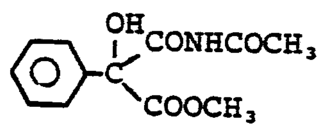

Zu einer Lösung aus 88,6 g Eisessig und 74 ml $H_2SO_4$ konz. wurde bei 40 - 50°C 94 g Phenylhydroxymalonsäuremethylesternitril zugetropft. Nach 1 Stunde wurde die Reaktionslösung auf Eiswasser gegeben und mit Methylenchlorid extrahiert. Nach dem Aufarbeiten der organischen Phase wurde der Rückstand aus i-Propanol umkristallisiert. Es blieben 50 g eines Gemisches aus Phenylhydroxymalonsäuremethylesteramid und Phenylhydroxymalonsäuremethylesteracetylamid im ungefähren Verhältnis 1:1, wie aus den spektroskopischen Daten zu entnehmen war.

Durch Behandeln des Substanzgemisches mit $NH_3$ in methanolischer Lösung bei 30 - 50°C während 2 Stunden wurde Phenylhydroxymalonsäurediamid vom Fp. 159°C erhalten.

Beispiel k

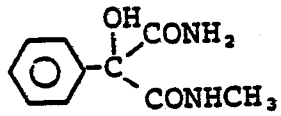

In eine Lösung von 42 g Phenylhydroxymalonsäuremethylesteramid in 400 ml Methanol wurde 4 Stunden bei Rückflußtemperatur Methylamin eingeleitet. Nachdem eingeengt worden war, wurden durch Umkristallisieren aus Ethanol 30 g Phenylhydroxymalonsäureamidmethylamid (Fp. = 128°C) erhalten.

Beispiel l

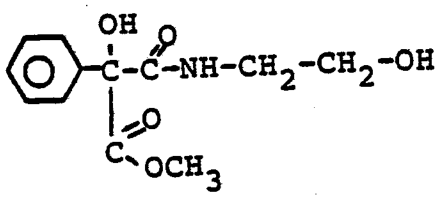

20,9 g Phenylhydroxymalonsäuremethylesteramid 6,1 g Ethanolamin und 60 ml Methanol wurden 8 Stunden am Rückfluß erhitzt. Nach dem Einengen (gegen Ende im Hochvakuum) blieben 24 g hochviskoser Rückstand, der spektroskopisch als das gewünschte Phenylhydroxymalonsäureamid-ß-hydroxyethylamid identifiziert wurde.

Beispiel m

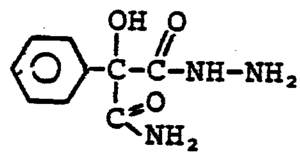

62,7 g Phenylhydroxymalonsäuremethylesteramid, 15 g Hydrazinhydrat und 200 ml Methanol wurden 4 Stunden am Rückfluß erhitzt. Es wurde bis 60°C Badtemperatur im Wasserstrahlvakuum eingeengt, mit Toluol versetzt und erneut eingeengt, gegen Ende im Hochvakuum. Es blieben 65 g glasiger Rückstand, der sich spektroskopisch als Phenylhydroxymalonsäureamidhydrazid erwies.

Beispiel n

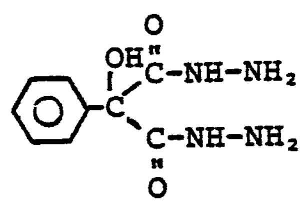

22,4 g Phenylhydroxymalonsäuredimethylester und 10 g Hydrazinhydrat wurden in 100 ml Methanol 5 Stunden am Rückfluß erhitzt. Anschließend wurde bei 50°C eingeengt, gegen Ende im Hochvakuum. Es blieben 24 g eines glasigen Rückstandes, der aus Wasser umkristallisiert wurde. Es wurden 14 g Phenylhydroxymalonsäurebishydrazid Fp. 159°C (Zersetzung) erhalten.

Beispiel o

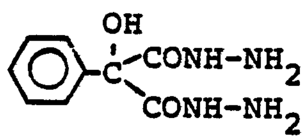

20,9 g Phenylhydroxymalonsäuremethylesteramid und 25 g Hydrazinhydrat wurden in 150 ml Ethanol 12 Stunden am Rückfluß erhitzt. Anschließend wurde eingeengt und aus 75 ml Wasser umkristallisiert. Es blieben 13 g Phenylhydroxymalonsäurebishydrazid vom Fp. 159°C (Zersetzung.

Beispiel p

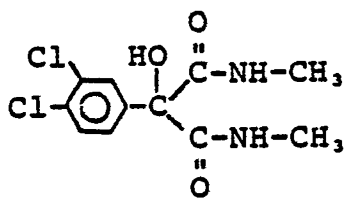

14,7 g 3,4-Dichlorphenylhydroxymalonsäuredimethylester wurden in 100 ml Methanol gelöst undbei 60°C 1 Stunde Methylamin eingeleitet. Es wurde völlig eingeengt und der kristalline Rückstand aus 100 ml Ethanol umkristallisiert. Es wurden 12 g 3,4-Dichlorphenylhydroxymalonsäuredimethylamid (Fp. = 158 - 60°C) erhalten.

Beispiel q

Analog zu obigem Verfahren wurde 3,4-Dichlorphenylhydroxymalonsäuredineopentylamid (Fp. = 94 - 95°C) erhalten.

Beispiel r

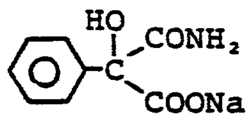

5,41 g Phenylhydroxymalonsäuremethylesteramid wurden mit 25,9 ml wäßriger 1n NaOH-Lösung 2,5 Stunden bei Raumtemperatur gerührt. Die Reaktionslösung wurde dabei fast homogen, der pH-Wert fiel auf 6. Nach Filtration wurde eingeengt, gegen Ende im Hochvakuum. IR, NMR und MS (FAB) bestätigten das Vorliegen des Mononatriumsalzes des Phenylhydroxymalonsäuremonoamids.

Beispiel s

Die freie Säure der Verbindung des Beispiels 32 wurde erhalten indem man das Salz des Bsp. 33 in Methylenchlorid suspendierte und mit HCl-Gas behandelte.

Beispiel t

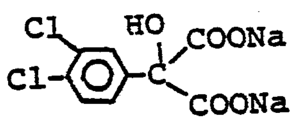

11,2 g 3,4-Dichlorphenylhydroxymalonsäuredimethylester wurden mit 76,6 ml wäßriger 1n NaOH-Lösung bei Raumtemperatur gerührt, bis die Lösung einen pH-Wert von 6 aufwies. Anschließend wurde abfiltriert und eingeengt.

Beispiel u

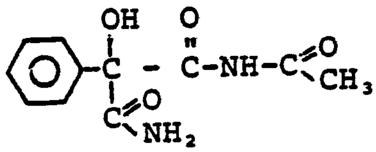

Zu einer Lösung aus 72 g Eisessig und 60 ml $H_2SO_4$ konz. wurden bei 40 - 50°C 92 g Phenyltrimethylsilyloxymalonsäuredinitril zugetropft. Nach 2 Stunden wurde die viskose Lösung auf Eis gegossen, gut durchgerührt und abgesaugt. Es blieben 53 g Rohprodukt, welches aus Wasser umkristallisiert wurde. Es blieben 40 g Phenylhydroxymalonsäureamidacetylamid; Fp. 210°C (Zersetzung).

Beispiel v

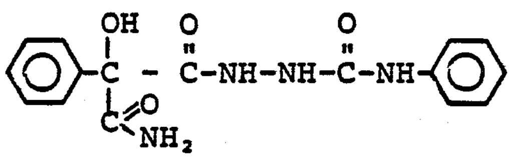

26,4 g Phenylhydroxymalonsäureamidhydrazid wurden in 50 ml abs. Dioxan gelöst und mit einer Lösung von 15 g Phenylisocyanat in 15 ml abs. Dioxan versetzt. Die Temperatur stieg dabei auf 40°C an und es fiel ein Niederschlag aus. Es wurde kalt abgesaugt und der Rückstand mit 500 ml Wasser ausgekocht. Es blieben 27 g nicht ganz sauberes Phenylhydroxymalonsäureamid-(phenylaminocarbonyl)-hydrazid (Fp. 199°C).

Beispiel w

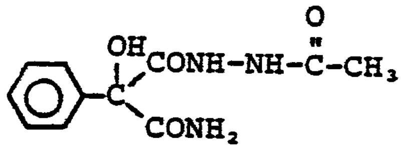

21,2 g Phenylhydroxymalonsäureamidhydrazid wurde in 100 ml Dioxan gelöst, 10,3 g Triethylamin zugefügt und 8 g Acetylchlorid zugetropft. Es wurde 1 Stunde bei Raumtemperatur nachgerührt, der Niederschlag abgesaugt und die Mutterlauge eingeengt. Danach blieben 28 g unsauberes Rohprodukt, welches in Essigsäureethylester aufgenommen und mit wenig Wasser gewaschen wurde. Nach entfernen des Lösungsmittels im Hochvakuum bei 60°C Badtemperatur blieb Phenylhydroxymalonsäureamid-acetylhydrazid als glasiger Rückstand.

Beispiel x

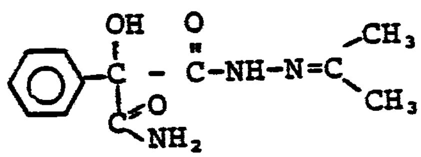

20 g Phenylhydroxymalonsäureamidhydrazid wurden in 100 ml Aceton 3 Stunden am Rückfluß erhitzt. Die Kristalle wurden abgesaugt und aus Ethanol umkristallisiert. Es fielen 15 g Phenylhydroxymalonsäureamid-(2-propyliden)-hydrazid (Fp. 199 - 201°C) an.

Beispiel y

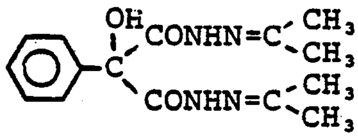

11,2 g Phenylhydroxymalonsäurebishydrazid wurde in 100 ml Aceton 4 Stunden am Rückfluß erhitzt. Anschließend wurde eingeengt, gegen Ende im Hochvakuum. Es blieben 13,8 g rohes Phenylhydroxymalonsäure-bis-(2-propyliden-hydrazid) als hochviskoser Rückstand.

Beispiel z

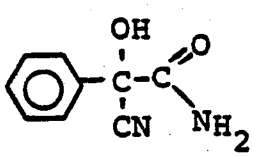

29,8 g Phenylglyoxysäureamid wurde in 30 ml Ethylenglykoldimethylether suspendiert, 0,5 ml Triethylamin zugegeben und mit 5,4 g Blausäure versetzt. Es entstand eine homogene Lösung. Nach Entfernen des Lösungsmittels blieb Phenylhydroxymalonsäureamidnitril zurück.

Beispiel A

Plutella-Test

Lösungsmittel: 3 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 2, 15, 24, 16, 1, 18, 19, 23, 3, 17, 22, 30, 31.

Beispiel B

Tetranychus-Test (resistent)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 14, 2, 16, 1, 18, 19, 23, 3, 17, 22, 30.

Beispiel C

Grenzkonzentrations-Test / Bodeninsekten

Testinsekt: Phorbia antiqua Madon (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 24.

Beispiel D

Grenzkonzentrations-Test / Wurzelsystemische Wirkung

Testinsekt: Phaedon cochleariae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 16, 2, 18, 23, 19.

Patentansprüche

1. Fünfgliedrige stickstoffhaltige Heterocyclen der Formel (I)

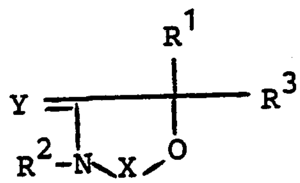

(I)

in welcher

$R^1$ für Alkyl, Alkenyl, Alkinyl, Cycloalkyl, Cycloalkenyl, Aryl, Heteroaryl, die gegebenenfalls substituiert sein können, steht

$R^2$ für Wasserstoff, ein Äquivalent eines anorganischen oder organischen Kations Trialkylsilyl sowie für Alkyl, Cycloalkyl, Alkenyl, Alkinyl, Aryl, Aralkyl, Alkylcarbonyl, Arylcarbonyl, Alkoxycarbonyl, Aryloxycarbonyl, Alkylsulfenyl, Arylsulfenyl, Alkylsulfonyl, Arylsulfonyl, Alkylaminosulfonyl, Dialkylaminosulfonyl, Arylaminosulfonyl, Arylalkylaminosulfonyl, die gegebenenfalls substituiert sein können, sowie für Reste der Formel

$$- CO - NR^5R^6$$

steht, wobei

$R^5$ und $R^6$ unabhängig voneinander für Wasserstoff, Alkyl, Cycloalkyl, Aryl, Alkylaminocarbonyl, Arylaminocarbonyl, Alkylcarbonyl, Arylcarbonyl, Alkoxycarbonyl, Aryloxycarbonyl, Alkylsulfonyl, Arylsulfonyl, wobei diese Reste gegebenenfalls substituiert sein können stehen,

$R^3$ steht für Alkyl, Alkenyl, Alkinyl, Cycloalkyl, Cycloalkenyl, Aryl, Heteroaryl, die gegebenenfalls substituiert sind; sowie für CN sowie für den Rest $-\overset{O(S)}{\overset{\|}{C}}-R^4$

$R^4$ steht für Amino, -OW wobei W für Wasserstoff, gegebenenfalls substituiertes Alkyl- oder Cycloalkyl, Aralkyl sowie für ein Äquivalent Alkali oder Erdalkali steht,

ferner steht $R^4$ für Alkylamino, Arylamino, Aralkylamino, Dialkylamino, Cycloalkylamino, Alkenylamino, Trialkylsilylamino, Trialkylsilylalkylamino, stickstoffhaltige gesättigte heterocyclische Reste die gegebenenfalls weitere Heteroatome enthalten, wobei die Reste gegebenenfalls substituiert sein können, ferner steht $R^4$ für Reste der Formel

$$-NHR^7$$

wobei

$R^7$ für Hydroxyl, Formyl, Alkylcarbonyl, Alkenylcarbonyl, Cycloalkenylcarbonyl, Arylcarbonyl, Amino, Alkylamino, Arylamino, Alkylarylamino, Dialkylamino, Alkylaminocarbonylamino ($-NH-\overset{O}{\overset{\|}{C}}-NH-Alkyl$), Alkylaminothiocarbonylamino ($-NH-\overset{S}{\overset{\|}{C}}-NH-Alkyl$), Arylaminocarbonylamino, Arylaminothiocarbonylamino, Alkylcarbonylamino ($-NH-\overset{O}{\overset{\|}{C}}-Alkyl$), Arylcarbonylamino, Alkylsulfonylaminocarbonylamino ($-NH-\overset{O}{\overset{\|}{C}}-NH-SO_2-Alkyl$), Arylsulfonylaminocarbonylamino, Alkylcarbonylaminocarbonylamino ($-NH-\overset{O}{\overset{\|}{C}}-NH-\overset{O}{\overset{\|}{C}}-Alkyl$), Arylcarbonylaminocarbonylamino wobei die Alkyl oder Arylreste gegebenenfalls substituiert sein können, steht,

$R^4$ steht ferner für Reste der Formel

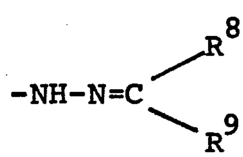

wobei

$R^8$ für Alkyl oder Aryl die gegebenenfalls substituiert sein können, steht und

$R^9$ für Wasserstoff oder Alkyl steht,

X steht für S, SO, $SO_2$, $>SiR^{10}R^{11}$, $>\overset{O}{\overset{\|}{P}}-O-R^{12}$,

wobei

$R^{10}$ für Alkyl steht,

$R^{11}$ für Alkyl steht,

$R^{12}$ für Alkyl oder Aryl steht, und

Y steht für O, S, $=N-R^{13}$

wobei

$R^{13}$ für Alkyl oder Aryl steht die gegebenenfalls substituiert sein können;

steht $R^1$ für Methyl oder Trifluormethyl darf $R^3$ nicht gleichzeitig für Methyl oder Trifluormethyl stehen.

Die fünfgliedrigen stickstoffhaltigen Heterocyclen der Formel I, liegen in Form der Gemische ihrer sterischen und optischen Isomere sowie in Form ihrer reinen Isomeren vor.

2. Verfahren zur Herstellung der fünfgliedrigen stickstoffhaltigen Heterocyclen der Formel I

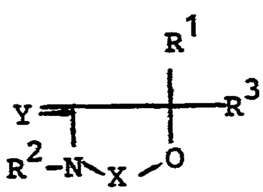

I

wobei

$R^1$ für Alkyl, Alkenyl, Alkinyl, Cycloalkyl, Cycloalkenyl, Aryl, Heteroalkyl, die gegebenenfalls substituiert sein können, steht

$R^2$ für Wasserstoff, ein Äquivalent eines anorganischen oder organischen Kations, Trialkylsilyl sowie für Alkyl, Cyloalkyl, Alkenyl, Alkinyl, Aryl, Aralkyl, Alkylcarbonyl, Arylcarbonyl, Alkoxycarbonyl, Aryloxycarbonyl, Alkylsulfenyl, Arylsulfenyl, Alkylsulfonyl, Arylsulfonyl, Alkylaminosulfonyl, Dialkylaminosulfonyl, Arylaminosulfonyl, Arylalkylaminosulfonyl, die gegebenenfalls substituiert sein können, sowie für Reste der Formel

$$-CO-NR^5R^6$$

steht, wobei

$R^5$ und $R^6$ unabhängig voneinander für Wasserstoff, Alkyl, Cycloalkyl, Aryl, Alkylaminocarbonyl, Arylaminocarbonyl, Alkylcarbonyl, Arylcarbonyl, Alkoxycarbonyl, Aryloxycarbonyl, Alkylsulfonyl, Arylsulfonyl, wobei diese Reste gegebenenfalls substituiert sein können stehen,

$R^3$ steht für Alkyl, Alkenyl, Alkinyl, Cycloalkyl, Cycloalkenyl, Aryl, Heteroaryl, die gegebenenfalls substituiert sind sowie für -CN sowie für

den Rest $-\overset{O(S)}{\overset{\|}{C}}-R4$,

$R^4$ steht für Amino, OW wobei W für Wasserstoff, gegebenenfalls substituiertes Alkyl- oder Cycloalkyl, Aralkyl sowie für ein Äquivalent Alkali oder Erdalkali steht,

ferner steht $R^4$ für Alkylamino, Arylamino, Aralkylamino, Dialkylamino, Cycloalkylamino, Alkenylamino, Trialkylsilylamino, Trialkylsilylalkylamino, stickstoffhaltige gesättigte heterocyclische Reste die gegebenenfalls weitere Heteroatome enthalten, wobei die Reste gegebenenfalls substituiert sein können, ferner steht $R^4$ für Reste der Formel

$$-NHR^7$$

wobei

$R^7$ für Hydroxyl, Formyl, Alkylcarbonyl, Alkenylcarbonyl, Cycloalkenylcarbonyl, Arylcarbonyl, Amino, Alkylamino, Arylamino, Alkylarylamino, Dialkylamino, Alkylaminocarbonylamino ($-NH-\overset{O}{\overset{\|}{C}}-NH-Alkyl$), Alkylaminothiocarbonylamino ($-NH-\overset{S}{\overset{\|}{C}}-NH-Alkyl$), Arylaminocarbonylamino, Arylaminothiocarbonylamino, Alkylcarbonylamino ($-NH-\overset{O}{\overset{\|}{C}}-Alkyl$), Arylcarbonylamino, Alkylsulfonylaminocarbonylamino ($-NH-\overset{O}{\overset{\|}{C}}-NH-SO_2-Alkyl$), Arylsulfonylamino-

carbonylamino, Alkylcarbonylaminocarbonylamino ($-NH-\overset{O}{\overset{\|}{C}}-NH-\overset{O}{\overset{\|}{C}}-$Alkyl), Arylcarbonylaminocarbonylamino, wobei die Alkyl- oder Arylreste gegebenenfalls substituiert sein können, steht,

$R^4$ steht ferner für Reste der Formel

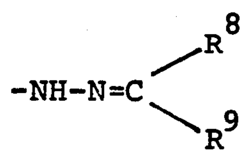

wobei

$R^8$ für Alkyl oder Aryl die gegebenenfalls substituiert sein können steht und

$R^9$ für Wasserstoff oder Alkyl steht,

X steht für S, SO, $SO_2$, $>SiR^{10}R^{11}$, $>\overset{O}{\overset{\|}{P}}-O-R^{12}$,

wobei

$R^{10}$ für Alkyl steht,

$R^{11}$ für Alkyl steht,

$R^{12}$ für Alkyl oder Aryl steht, und

Y steht für O, S, $=N-R^{13}$

wobei

$R^{13}$ für Alkyl oder Aryl steht die gegebenenfalls substituiert sein können;

steht $R^1$ für Methyl oder Trifluormethyl darf $R^3$ nicht gleichzeitig für Methyl oder Trifluormethyl stehen, dadurch gekennzeichnet, daß man

a) für den Fall, daß Verbindungen der Formel I erhalten werden sollen,

in welcher X für SO, S, $SiR^{10}R^{11}$ steht, Verbindungen der Formel II

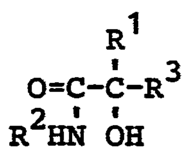

II

in welcher

$R^1$, $R^2$, $R^3$, die oben angegebene Bedeutung haben

mit Verbindungen der Formel III

$X^1Hal_2$ III

in welcher

Hal für Halogen steht und

$X^1$ für SO, $>SiR^{10}R^{11}$, S steht,

umsetzt, und die entstehenden Verbindungen gegebenenfalls zur Salzbildung mit einer Base behandelt, oder

b) Verbindungen der Formel IV

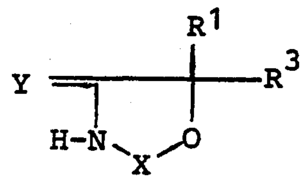

IV

in welcher

$R^1$, $R^3$, X und Y die oben angegebene Bedeutung haben

mit Verbindungen der Formel V

$R^{14}$-Z V

in welcher

$R^{14}$ für Trialkylsilyl, Alkyl, Cycloalkyl, Alkenyl, Alkinyl, Aralkyl, Aryl, Alkylcarbonyl, Arylcarbonyl, Alkoxycarbonyl, Aryloxycarbonyl, Alkylsulfenyl, Arylsulfenyl, Alkylsulfonyl, Arylsulfonyl, Alkylaminosulfonyl, Dialkylaminosulfonyl, Arylaminosulfonyl, Arylalkylaminosulfonyl, die gegebenenfalls substituiert sein können, sowie für Reste der Formel

$$-CO-NR^5R^6$$

steht, wobei

$R^5$ und $R^6$ unabhängig voneinander für Wasserstoff, Alkyl, Cycloalkyl, Aryl, Alkylaminocarbonyl, Arylaminocarbonyl, Alkylcarbonyl, Arylcarbonyl, Alkoxycarbonyl, Aryloxycarbonyl, Alkylsulfonyl, Arylsulfonyl, wobei diese Reste gegebenenfalls substituiert sein können stehen,

Z für eine gegenüber einem aciden Wasserstoffatom reaktive Gruppe steht,

umsetzt, oder

c) für den Fall, daß Verbindungen der Formel I erhalten werden sollen, in denen X für $>SiR^{10}R^{11}$ steht, man Verbindungen der Formel II

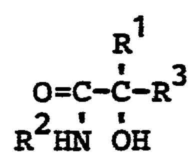

II

in welcher

$R^1$, $R^2$, $R^3$ die oben angegebene Bedeutung haben,

$R^3$ jedoch nicht für -COOH stehen darf,

mit Verbindungen der Formel IX

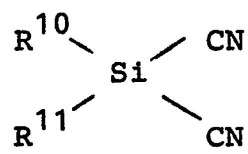

IX

in welcher

$R^{10}$ und $R^{11}$ die oben angegebene Bedeutung haben

umsetzt und die entstehenden Verbindungen gegebenenfalls zur Salzbildung mit einer Base behandelt, oder

d) für den Fall, daß Verbindungen der Formel I erhalten werden sollen, in denen X für $> \overset{\overset{O}{\|}}{P}-OR^{12}$ und $R^2$ für den Rest $R^{12}$ steht

man Verbindungen der Formel X

$$R^1-\overset{\overset{O}{\|}}{C}-\overset{\overset{O}{\|}}{C}-R^{16} \qquad X$$

in welcher

$R^1$ die oben angegebene Bedeutung besitzt und

$R^{16}$ für Amino, Alkylamino, Arylamino, Aralkylamino, Dialkylamino, Cycloalkylamino, Alkenylamino, Trialkylsilylamino, Trialkylsilylalkylamino, stickstoffhaltige gesättigte heterocyclische Reste die gegebenenfalls weitere Heteroatome enthalten, wobei die Reste gegebenenfalls substituiert sein können, ferner steht $R^{16}$ für Reste der Formel

$$-NHR^7$$

wobei

$R^7$ für Hydroxyl, Formyl, Alkylcarbonyl, Alkenylcarbonyl, Cycloalkenylcarbonyl, Arylcarbonyl, Amino, Alkylamino, Arylamino, Alkylarylamino, Dialkylamino, Alkylaminocarbonylamino

$$(-NH-\overset{O}{\overset{\|}{C}}-NH-Alkyl)$$

, Alkylaminothiocarbonylamino

$$(-NH-\overset{S}{\overset{\|}{C}}-NH-Alkyl)$$

, Arylaminocar-

bonylamino, Arylaminothiocarbonylamino, Alkylcarbonylamino

O
‖
(-NH-C-Alkyl), Arylcarbonylamino, Alkylsulfonylaminocarbonylamino

C
‖
(-NH-C-NH-$SO_2$-Alkyl), Arylsulfonylaminocarbonylamino, Alkylcarbonylaminocarbonyl-

O ‖ O ‖
amino (-NH-C-NH-C-Alkyl), Arylcarbonylaminocarbonylamino wobei die Alkyl oder Arylreste gegebenenfalls substituiert sein können, steht,

$R^{16}$ steht ferner für Reste der Formel

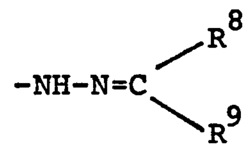

wobei

$R^8$ für Alkyl oder Aryl die gegebenenfalls substituiert sein können, steht und

$R^9$ für Wasserstoff oder Alkyl steht,

mit Verbindungen der Formel XI

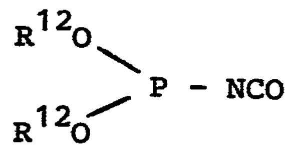

XI

in welcher

$R^{12}$ die oben angegebene Bedeudeutung hat,

umsetzt oder

e) für den Fall, daß Verbindungen der Formel I erhalten werden sollen, in welcher X für $SO_2$ steht, man Verbindungen der Formel I in welcher X für SO steht mit Oxidationsmitteln wie Peroxiden oder Persäuren umsetzt.

3. Verbindungen der Formel I in welcher

$R^1$ für $C1-7$-Alkyl, $C_{2-7}$-Alkenyl, $C_{3-7}$-Cycloalkyl, $C_{5-8}$-Cycloalkenyl steht, die gegebenenfalls gleich oder verschieden durch einen oder mehrere der folgenden Reste substituiert sein können:

Halogen, insbesondere Fluor, Chlor, Brom, $C_{1-4}$-Alkoxy insbesondere Methoxy oder

Ethoxy, Carboxyl, Carbalkoxy insbesondere Methoxycarbonyl oder Ethoxycarbonyl, Phenyl, Phenoxy, Thiophenyl wobei die Phenylringe durch Halogen oder Alkyl substituiert sein können;

$R^1$ steht ferner für Phenyl das gegebenenfalls gleich oder verschieden durch einen oder mehrere der folgenden Reste substituiert sein kann: Halogen, insbesondere Chlor, Brom, Fluor, Nitro, Amino, OH, CN, $C_{1-4}$-Alkyl insbesondere Methyl, $C_{1-4}$-Halogenalkyl insbesondere Trifluormethyl, Trichlormethyl, Pentafluorethyl, $C_{1-4}$-Alkoxy, Methylendioxy, Ethylendioxy, $C_{1-4}$-Halogenalkoxy, insbesondere Trifluormethoxy, Pentafluorethoxy, Difluormethylendioxy, halogensubstituiertes Ethylendioxy, $C_{1-4}$-Alkylthio, $C_{1-4}$-Halogenalkylthio insbesondere Trifluormethylthio, $C_{2-8}$-Alkoxyalkyl, $C_{2-8}$-Halogenalkoxyalkyl, $C_{1-4}$-Alkylsulfonyl insbesondere Methylsulfonyl, $C_{1-4}$-Halogenalkylsulfonyl, Carboxyl, Carbalkoxy insbesondere Methoxycarbonyl, sowie für den Rest $C_{1-4}$-Alkoxy-N=CH-, insbesondere $CH_3$-O-N=CH-, ferner für Phenyl, Phenyloxy, Thiophenyl die gegebenenfalls durch Halogen oder $C_{1-4}$-Alkyl substituiert sein können, sowie für Carboxylalkoxy mit 2 - 4 C-Atomen wie Carboxymethoxy,

$R^1$ steht ferner für Heteroaryl wie Pyridinyl, Pyrimidinyl, Triazinyl, Isoxazolyl, Thiazolyl, Oxadiazolyl, Imidazolyl, Triazolyl, Furanyl, Thiophenyl, die gegebenenfalls ein oder mehrfach gleich oder verschieden durch Halogen insbesondere Chlor, $C_{1-4}$-Alkyl, insbesondere Methyl, Ethyl, $C_{1-4}$-Alkoxy, insbesondere Methoxy, Ethoxy substituiert sein können,

$R^2$ steht für Wasserstoff, ein Äquivalent eines Alkali, Erdalkali oder gegebenenfalls substituierten Ammonium-Kations, Trialkylsilyl mit 1 - 4 C-Atomen im Alkylteil, $C_{1-4}$-Alkyl, $C_{1-4}$-Alkylcarbonyl, Phenyl, Benzoyl, die gegebenenfalls gleich oder verschieden durch einen oder mehrere der folgenden Reste (A) substituiert sein kann (A) steht für Halogen, insbesondere Chlor, Brom, Fluor, Nitro, Amino, CN, $C_{1-4}$-Alkyl insbesondere Methyl, $C_{1-4}$-Halogenalkyl insbesondere Trifluormethyl, Pentafluorethyl, $C_{1-4}$Alkoxy, $C_{1-4}$-Halogenalkoxy, insbesondere Trifluormethoxy, Pentafluorethoxy, Methylendioxy, Ethylendioxy, Difluormethylendioxy, halogensubstituierte Ethylendioxy wie Trifluorethylendioxy, $C_{1-4}$-Alkylthio, $C_{1-4}$-Halogenalkylthio insbesondere Trifluormethylthio, $C_{2-8}$-Alkoxyalkyl, $C_{2-8}$-Halogen-

alkoxyalkyl, $C_{1-5}$-Alkylsulfenyl, Phenylsulfenyl die gegebenenfalls substituiert sein können, $C_{1-4}$-Alkylsulfonyl insbesondere Methylsulfonyl, $C_{1-4}$-Halogenalkylsulfonyl, Carbalkoxy insbesondere Methoxycarbonyl, sowie für den Rest $C_{1-4}$-Alkoxy-N=CH-, insbesondere $CH_3$-O-N=CH-, ferner für Phenyl, Phenyloxy, Thiophenyl die gegebenenfalls durch Halogen oder $C_{1-4}$-Alkyl substituiert sein können, sowie für Carboxyalkoxy mit 2 - 4 C-Atomen, wie Carboxymethoxy;

$R^2$ steht ferner für $C_{1-4}$-Alkoxycarbonyl, Phenoxycarbonyl, das gegebenenfalls durch einen oder mehrere der Reste (A) substituiert sein kann, $C_{1-4}$-Alkylsulfonyl, Phenylsulfonyl das gegebenenfalls durch einen oder mehrere der Reste (A) substituiert sein kann, $C_{1-4}$-Alkylaminosulfonyl, $C_{1-5}$-Alkylsulfenyl, Phenylsulfenyl die gegebenenfalls substituiert sein können, Di-$C_{1-4}$-alkylaminosulfonyl, Phenylaminosulfonyl das gegebenenfalls durch einen oder mehrere der Reste (A) substituiert sein kann, Phenyl-$C_{1-4}$-alkyl-aminosulfonyl sowie für Reste der Formel

$$-\overset{O}{\overset{\|}{C}}-NR^5R^6$$

wobei

$R^5$ und $R^6$ unabhängig voneinander für Wasserstoff, $C_{1-20}$-Alkyl, $C_{3-6}$-Cycloalkyl, Phenyl das gegebenenfalls durch einen oder mehrere der Reste (A) substituiert sein kann, Phenylcarbonyl, das gegebenenfalls durch einen oder mehrere der Reste (A) substituiert sein kann, $C_{1-10}$-Alkylcarbonyl, $C_{1-4}$-Alkylaminocarbonyl, $C_{1-10}$-Alkoxycarbonyl, Phenylaminocarbonyl, Phenylsulfonyl die gegebenenfalls durch einen oder mehrere der Reste (A) substituiert sein können, stehen,

$R^3$ steht für die bei $R^1$ angegebenen Reste sowie außerdem für CN oder den Rest $COR^4$, $R^4$ steht für Amino, $C_{1-8}$-Alkylamino, Di-$C_{1-8}$-alkylamino, die gegebenenfalls durch Hydroxyl, $C_{1-4}$-Alkoxy substituiert sein können, Phenylamino das gegebenenfalls durch einen oder mehrere der oben angegebenen Reste (A) substituiert sein kann, $C_{5-6}$-Cycloalkylamino, einen gesättigten Heterocyclus mit 5 - 6 Ring C-Atomen wie Morpholin, Tri-$C_{1-4}$-alkylsilylamino, sowie für Reste der Formel

$$-NHR^7$$

wobei

$R^7$ für $C_{1-4}$-Alkylcarbonyl, $C_{2-4}$-Alkenylcarbonyl,

$C_{5-8}$-Cycloalkenylcarbonyl, $C_{1-4}$-Alkylamino , Phenylamino das gegebenenfalls durch einen oder mehrere der oben angegebenen Reste (A) subsituiert sein kann, $C_{1-4}$-Alkylaminocarbonylamino ($-NH-\overset{O}{\overset{\|}{C}}-NH-Alkyl$), Phenylaminocarbonylamino ($-NH-\overset{O}{\overset{\|}{C}}-NH-Phenyl$) das gegebenenfalls durch einen oder mehrere der Reste (A) substituiert sein kann, $C_{1-4}$-Alkylcarbonylamino ($-NH-\overset{O}{\overset{\|}{C}}-Alkyl$), Benzoylamino das gegebenenfalls durch einen oder mehrere der Reste (A) substituiert sein kann, steht;

$R^4$ steht ferner für einen Rest der Formel

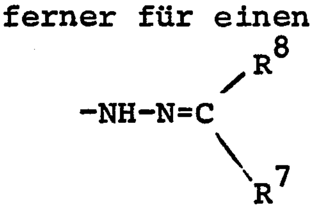

wobei

$R^8$ für $C_{1-4}$-Alkyl insbesondere Methyl, Phenyl das gegebenenfalls durch einen oder mehrere der Reste (A) substituiert sein kann, steht,

$R^9$ für Wasserstoff oder $C_{1-4}$-Alkyl steht, und

Y steht für O und

X steht für SO und $SiR^{10}R^{11}$

wobei

$R^{10}$ und $R^{11}$ für $C_{1-4}$-Alkyl insbesondere für Methyl stehen.

4. Verbindungen der Formel I

in welcher

$R^1$ für $C_{1-6}$-Alkyl das gegebenenfalls durch Halogen, $C_{1-4}$-Alkoxycarbonyl oder Phenoxy substituiert ist, wobei der Phenoxyrest gegebenenfalls durch Halogen oder Methyl substituiert ist, $C_{5-6}$-Cycloalkyl, für gegebenenfalls durch Carboxyl substituiertes $C_{2-4}$-Alkenyl, ferner für Phenyl, das gegebenenfalls durch Halogen, $C_{1-4}$-Alkyl, $C_{1-4}$-Alkoxy, $C_{1-4}$-Halogenalkoxy, $C_{1-4}$-Halogenalkyl, $-NH_2$, $CH_3O-N=CH-$, oder Nitro substituiert ist, steht und

$R^2$ für Wasserstoff, $C_{1-6}$-Alkyl, $C_{2-4}$-Alkenyl, $C_{2-4}$-Alkinyl oder für ein Äquivalent eines Kations aus der Reihe Natrium, Kalium,

Calcium, Ammonium, Tri-($C_{1-4}$-alkyl)-ammonium, Morpholinyl, ferner für $C_{1-4}$-Alkylsulfenyl, $C_{1-4}$-Halogenalkylsulfenyl, Phenylsulfenyl das gegebenenfalls durch Halogen oder $C_{1-4}$-Alkyl substituiert ist, Phenyl, das gegebenenfalls durch Halogen oder $C_{1-4}$-Alkyl substituiert ist, steht,

$R^3$ für $C_{1-4}$-Alkyl, $C_{1-4}$-Halogenalkyl, sowie für den Rest $COR^4$ wobei $R^4$ bevorzugt für Amino, $C_{1-4}$-Alkylamino, $C_{1-4}$-Alkoxy steht, und

X für SO, $Si(CH_3)_2$ steht und

Y für O steht.

5. Verbindungen der Formel I in welcher

$R^1$ für $C_{1-4}$-Alkyl, Methylcarbonyl-$C_{1-4}$-alkoxy, $C_{1-4}$-Halogenalkyl, Phenoxymethyl das gegebenenfalls durch Chlor, Fluor, Methyl substituiert ist, Phenyl das gegebenenfalls ein bis dreifach gleich oder verschieden durch Halogen, $C_{1-4}$-Alkyl, Nitro, $C_{1-4}$-Alkoxy substituiert ist, steht,

$R^2$ für Wasserstoff, $Na^{\oplus}$, $C_{1-4}$-Alkyl, $C_{1-4}$-Halogenalkyl, gegebenenfalls halogensubstituiertes $C_{1-4}$-Alkylsulfenyl, $C_{1-4}$-Alkylcarbonyl, gegebenenfalls durch Halogen substituiertes Phenyl, Phenylcarbonyl oder Phenylsulfenyl, $C_{1-4}$-Alkenyl, $C_{1-4}$-Alkinyl, Trialkylsilyl, sowie für $-\overset{O}{\overset{\|}{C}}-NR^5R^6$ steht wobei $R^5$ für Wasserstoff steht und $R^6$ für gegebenenfalls

0126235

durch Halogen substituiertes Phenyl oder Phenylcarbonyl steht,

$R^3$ für $C_{1-4}$-Alkyl, $C_{1-4}$-Halogenalkyl, Phenyl, das gegebenenfalls durch Chlor, Brom, Fluor substituiert ist sowie für den Rest $COR^4$ steht wobei $R^4$ für Amino steht,

X für SO, und $Si(CH_3)_2$ steht und

Y für O steht.

6. Verbindungen der Formel I

in welcher

$R^1$ für t-Butyl, Chlormethyl, $C_{1-4}$-Alkoxycarbonylmethyl, gegebenenfalls durch Chlor oder Fluorsubstituiert Phenoxymethyl sowie für Phenyl steht das gegebenenfalls ein oder mehrfach durch Chlor, Methyl, Nitro, Trifluormethyl substituiert ist und

$R^2$ für Wasserstoff, Natrium Methyl, Ethyl, Propargyl, Trihalogenmethylsulfenyl, Tri($C_{1-4}$-alkyl)-ammonium, Morpholinyl steht,

$R^3$ für Phenyl, t-Butyl, oder $COR^4$ steht wobei $R^4$ für Amino steht,

steht.

7. Schädlingsbekämpfungsmittel, gekennzeichnet durch einen Gehalt an mindestens einem fünfgliedrigen stickstoffhaltigen Heterocyclen der Formel (I), gemäß Anspruch 1.

8. Verwendung von fünfgliedrigen stickstoffhaltigen Heterocyclen der Formel (I) gemäß Anspruch 1 zur Bekämpfung von Schädlingen.

9. Verfahren zur Herstellung von Schädlingsbekämpfungsmitteln, dadurch gekennzeichnet, daß man fünfgliedrigen stickstoffhaltigen Heterocyclen der Formel (I) gemäß Anspruch 1 mit Streckmitteln und/oder oberflächenaktiven Mitteln vermischt.

Europäisches Patentamt

**EUROPÄISCHER RECHERCHENBERICHT**

0 126235

Nummer der Anmeldung

EP 84 10 3069

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | FR-A-2 315 922 (ROUSSEL-UCLAF) * Anspruch 1 * --- | 1 | C 07 D 291/04<br>C 07 F 7/10<br>C 07 F 9/65<br>A 01 N 43/00<br>A 01 N 55/00<br>A 01 N 57/36 |
| X | Chemical Abstracts Band 83, Nr. 21, 24. November 1975, Columbus, Ohio, USA; V.V. DOVLATYAN et al. "Synthesis of 2,2-dioxo-4-chloro-1,2,3-oxathiazole-delta3 and its 5,5-dimethyl derivative and some of their transformations", Seiten 566-567, Spalten 2, 1; Abstract Nr. 178938p & Arm. Khim. Zh., Band 28, Nr. 4, 1975, Seiten 311-316 --- | 1 | |
| X,D | Chemical Abstracts Band 86, Nr. 15, 11. April 1977, Columbus, Ohio, USA; V.V. DOVLATYAN et al. "Reaction of thionylanilines with cyanohydrins and alpha-aminoisobutyronitrile", Seite 502, Spalte 2, Abstract Nr. 106492c & Arm. Khim. Zh., Band 29, Nr. 9, 1976, Seiten 764 -770 --- | 1 | **RECHERCHIERTE SACHGEBIETE (Int. Cl. 3)**<br>A 01 N 43/00<br>A 01 N 43/82<br>A 01 N 55/00<br>A 01 N 57/36<br>C 07 D 291/04<br>C 07 F 7/08<br>C 07 F 7/10<br>C 07 F 9/65 |
| X | JOURNAL OF ORGANIC CHEMISTRY, Band 42, Nr. 6, 1977, Columbus, USA; J.A. DEYRUP et al. "1-Oxo-1,2,5-thiadiazolidin-3-ones. A structural reassignment", Seiten 1015-1018 * Formel 3 * --- -/- | 1 | |

Der vorliegende Recherchenbericht wurde für alle Patentansprüche erstellt.

| Recherchenort | Abschlußdatum der Recherche | Prüfer |
|---|---|---|
| BERLIN | 02-07-1984 | KNAACK M |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82

Europäisches Patentamt

# EUROPÄISCHER RECHERCHENBERICHT

0126235 Nummer der Anmeldung

EP 84 10 3069

Seite 2

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| A | Chemical Abstracts Band 83, Nr. 7, 18. August 1975, Columbus, Ohio, USA; I.V. KONOVALOVA et al. "Reactions of dialkylphosphorous acid monoisocyanates with alpha-oxocarboxylic acid nitriles", Seite 495, Spalten 1, 2, Abstract Nr. 58723t & Zh. Obshch. Khim., Band 45, Nr. 5, 1975, Seiten 1003-1005<br>--- | 1 | |
| A | ZEITSCHRIFT FÜR ANORGANISCHE UND ALLGEMEINE CHEMIE, Band 406, 1974, Leipzig, DDR; H. STEINBEISSER et al. "Über Reaktionen des Bis(trifluormethyl)diazomethan mit Sulfonylisocyanaten", Seiten 299-306; Formel 5<br>--- | 1 | RECHERCHIERTE SACHGEBIETE (Int. Cl. 3) |
| A | EP-A-0 028 752 (BAYER)<br>* Ansprüche 1, 5 *<br>----- | 7 | |

Der vorliegende Recherchenbericht wurde für alle Patentansprüche erstellt.

| Recherchenort | Abschlußdatum der Recherche | Prüfer |
|---|---|---|
| BERLIN | 02-07-1984 | KNAACK M |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82